(19) Europäisches Patentamt
European Patent Office
Office européen des brevets

(11) Veröffentlichungsnummer: **0 072 932**
**B1**

(12) **EUROPÄISCHE PATENTSCHRIFT**

(45) Veröffentlichungstag der Patentschrift: **17.01.90**

(21) Anmeldenummer: **82106766.7**

(22) Anmeldetag: **27.07.82**

(51) Int. Cl.⁴: **C 07 D 241/08**, C 07 D 401/04, C 07 D 403/04, C 07 D 409/04, C 07 D 409/14, A 61 K 31/495, A 61 K 31/535

(54) **Neue Piperazinone, ihre Herstellung und Verwendung.**

(30) Priorität: **20.08.81 DE 3132882**

(43) Veröffentlichungstag der Anmeldung: **02.03.83 Patentblatt 83/09**

(45) Bekanntmachung des Hinweises auf die Patenterteilung: **17.01.90 Patentblatt 90/03**

(84) Bennante Vertragsstaaten: **AT BE CH DE FR GB IT LI NL SE**

(56) Entgegenhaltungen:
**AT-B-356 122**
**DE-A-2 438 619**
**DE-B-1 197 089**
**US-A-3 935 214**

**Chemical Abstracts Band 87, Nr. 7, 15. August 1977, Columbus, Ohio, USA V.M. DIXIT et al. "Agents acting on the central nervous system: Part XXV. 2-Substituted 1,2,3,4,6,7,8,12b-octahydropyrazino (2,1-a) (2)benzazepines" Seite 466, Spalte 2, Abstract Nr. 53214a**

**Chemical Abstracts Band 71, Nr. 19, 10. November 1969, Columbus, Ohio, USA A. SUT et al. "N-Monoalkylation of some 2-oxo- and 2,5-dioxopiperazines" Seite 385, Spalten 1, 2, Abstract Nr. 91 427z**

(73) Patentinhaber: **CASSELLA Aktiengesellschaft**
**Hanauer Landstrasse 526**
**D-6000 Frankfurt am Main 61 (DE)**

(72) Erfinder: **Beyerle, Rudi, Dr.**
**An der Pfaffenmauer 44**
**D-6000 Frankfurt a. Main 60 (DE)**
Erfinder: **Bender, Heinz, Dr.**
**Kirchgasse 6**
**D-6000 Frankfurt a. Main (DE)**
Erfinder: **Schindler, Ursula, Dr.**
**Reinhardswaldweg 1**
**D-6082 Mörfelden-Walldorf (DE)**
Erfinder: **Nitz, Rolf-Eberhard, Dr.**
**Heinrich-Bingemerweg 64**
**D-6000 Frankfurt a. Main 60 (DE)**
Erfinder: **Martorana, Piero Anton, Dr.**
**Kaiser-Friedrich Promenade 108**
**D-6380 Bad Homburg vor der Höh (DE)**

(74) Vertreter: **Urbach, Hans-Georg, Dr.**
**Hanauer Landstrasse 526**
**D-6000 Frankfurt am Main 61 (DE)**

**EP 0 072 932 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die vorliegende Erfindung betrifft substituierte Piperazin-2-one der allgemeinen Formel I

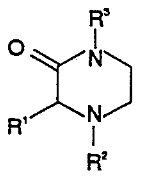

(I)

worin

$R^1$ durch Alkoxy mit 1 bis 4 C-Atomen, Fluor, Alkoxyalkyl mit insgesamt 2 bis 6 C-Atomen oder Dialkylaminoalkoxy mit insgesamt 4 bis 8 C-Atomen substituiertes Phenyl; 2-Thienyl; 3-Pyridyl oder einen Rest der Formel II

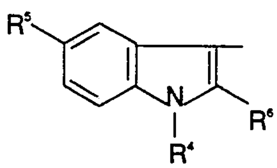

(II);

$R^2$ Wasserstoff; Alkyl mit 1 bis 4 C-Atomen, gegebenenfalls substituiert durch Alkoxycarbonyl mit insgesamt 2 bis 5 Kohlenstoffatomen, oder durch Aminocarbonyl, wobei die Aminogruppe des Aminocarbonylrestes ihrerseits 1- oder 2-fach durch Methyl oder Ethyl substituiert sein kann, oder Glied eines Piperidin-, Piperazin- oder Morpholinringes ist; Phenylalkyl mit 1 bis 3 C-Atomen im Alkylrest, gegebenenfalls substituiert im Phenylkern durch Chlor oder Alkoxy mit 1 oder 2 C-Atomen oder Hydroxy; Alkanoyl mit 1 bis 4 C-Atomen, das gegebenenfalls durch Chlor substituiert ist; Nicotinoyl; Benzoyl, gegebenenfalls substituiert durch Alkoxy mit 1 oder 2 C-Atomen, Nitro oder Amino; Aminosulfonyl; Aminosulfonyl, das am N durch 1 oder 2 Alkylgruppen mit je 1 bis 4 C-Atomen substituiert ist, oder Aminosulfonyl, dessen Aminogruppe Glied eines Piperidin-, Piperazin-, Morpholin- oder 4-Alkyl-piperazin-Ringes ist, wobei der 4-Alkyl-piperazinring 1 bis 4 C-Atome im Alkylrest aufweist; einen Rest der Formel III

$-C-CH_2-R^7$
||
O

(III);

einen Rest der Formel IV

$-C-NH-R^8$
||
O

(IV);

einen Rest der Formel V

$-CH_2-CH_2-OR^9$ (V);

$R^3$ Wasserstoff; Alkyl mit 1 bis 4 C-Atomen; Alkoxycarbonylmethyl mit insgesamt 3 bis 6 C-Atomen; Aminocarbonylmethyl; N-mono- oder disubstituiertes Aminocarbonylmethyl mit insgesamt 3 bis 5 C-Atomen oder Diethylaminocarbonylmethyl; oder eine Gruppe der Formel VII

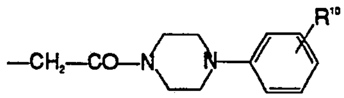

(VII);

$R^4$ Wasserstoff; Alkyl mit 1 bis 4 C-Atomen; Phenalkyl mit 1 oder 2 C-Atomen im Alkylrest, das gegebenenfalls durch Chlor substituiert ist;
$R^5$ Wasserstoff oder Alkoxy mit 1 bis 4 C-Atomen;
$R^6$ Wasserstoff oder Phenyl;
$R^7$ Amino; Piperidin-1-yl; Piperazin-1-yl; Morpholin-1-yl oder 4-Alkylpiperazin-1-yl mit 1 bis 4 C-Atomen im Alkylrest;
$R^8$ Wasserstoff; gegebenenfalls durch Chlor substituiertes Phenyl;
$R^9$ Wasserstoff; Phenoxyacetyl oder einen Rest der Formel VI

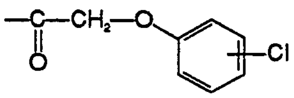

(VI)

$R^{10}$ Wasserstoff oder Alkoxy mit 1 bis 4 C-Atomen bedeuten und ihre physiologisch verträglichen Säureadditionssalze.

Die Erfindung betrifft weiterhin substituierte Piperazinone der Formel Ia

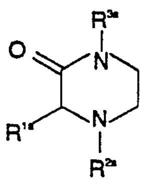

(Ia)

worin

$R^{1a}$ 2-Furyl;
$R^{2a}$ Wasserstoff, 2-Hydroxyethyl oder 2-Chlorbenzyl und
$R^{3a}$ Wasserstoff

bedeuten, sowie substituierte Piperazinone der Formel Ib

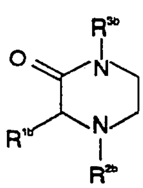

(Ib)

worin

$R^{1b}$ 2-Thienyl,
$R^{2b}$ Allyl, oder Propargyl und
$R^{3b}$ Wasserstoff

bedeuten, und 3-(4-(2-Methoxy-ethoxy)-phenyl)-piperazin-2-on.

Die Erfindung betrifft auch ein Verfahren zur Herstellung der Verbindungen der allgemeinen Formel I.

Ein für $R^1$ stehender Phenylrest kann bis zu 3 Substituenten aufweisen. Bei Einfachsubstitution des Phenylrestes kann der Substituent in o-, m- oder p-Stellung zum Piperazinonring stehen. Bei Zweifachsubstitution des Phenylrings sind die Kombinationen 2,4-, 3,4-, 2,5- und 3,5- bevorzugt. Bei Dreifachsubstitution sind die bevorzugten Substitutionsorte 2,4,6- und 3,4,5-. Zweckmäßigerweise enthält ein für R stehender Phenylrest maximal einen voluminöseren Substituenten, wie beispielsweise eine Dialkylaminoalkoxygruppe. Dagegen können bis zu drei der kleineren Substituenten Fluor oder insbesondere Methoxy an den Phenylrest gebunden sein.

Ein für $R^4$ stehender Phenalkylrest kann durch Chlor, insbesondere durch 1 oder 2 Chloratome, die entweder in o-, m- oder p-, bzw. in 2,3-, 2,5-, 3,4- oder 3,5-Stellung stehen können, substituiert sein.

Beispiele für Substituenten, die für $R^1$ stehen können, sind Phenyl, o-, m-, p-Methoxyphenyl, o-, m-, p-Ethoxyphenyl, 2,4-, 3,4- oder 3,5-Dimethoxyphenyl, 2,4,5-, 2,4,6- oder 3,4,5-Trimethoxyphenyl, o-, m-, p-Fluorphenyl, 2,4-, 2,5-, 3,4- oder 3,5-Difluorphenyl, o-, m-, p-Methoxymethylphenyl, o-, m-, p-Ethoxymethylphenyl, o-, m-, p-Propoxymethylphenyl, o-, m-, p-Butoxymethylphenyl, o-, m-, p-Methoxyethylphenyl, o-, m-, p-Ethoxyethylphenyl, o-, m-, p-Propoxyethylphenyl, o-, m-, p-Butoxyethylphenyl, o-, m-, p-Methoxypropylphenyl, o-, m-, p-Ethoxypropylphenyl, o-, m-, p-Propoxypropylphenyl, 2,4-, 3,4-, 2,5- oder 3,5-Bis-methoxymethylphenyl, 2,4-, 3,4-, 2,5- oder 3,5-Bis-methoxyethylphenyl, 2,4-, 3,4-, 2,5- oder 3,5-Bis-ethoxymethylphenyl, o-, m-, p-Dimethylaminoethoxyphenyl, o-, m-, p-Diethylaminoethoxyphenyl, o-, m-, p-Dipropylaminoethoxyphenyl, o-, m-, p-Dimethylaminopropoxyphenyl, o-, m-, p-Diethylaminopropoxyphenyl, o-, m-, p-Diethylaminobutoxyphenyl, o-, m-, p-Dimethylaminobutoxyphenyl, 2-Thienyl, 3-Pyridyl der 1-Indol-3-yl-rest der Formel II, in dem $R^4$ Wasserstoff, Methyl, Ethyl, Benzyl, o-, m-, p-Chlorbenzyl, Phenethyl, o-, m-, p-Chlorphenethyl bedeutet, in dem $R^5$ Wasserstoff, Methoxy oder Ethoxy bedeutet und in dem $R^6$ Wasserstoff oder Phenyl bedeutet.

Beispiele für besonders bevorzugte für $R^1$ stehende Reste sind: Phenyl, o-, m-, p-Methoxyphenyl, 2,4-, 3,4 oder 3,5-Dimethoxyphenyl, 3,4,5-Trimethoxyphenyl, o-, m-, p-Fluorphenyl, o-, m-, p-Methoxymethylphenyl, o-, m-, p-Ethoxymethylphenyl, o-, m-, p-Methoxyethylphenyl, o-, m-, p-Ethoxyethylphenyl, o-, m-, p-Dimethylaminoethoxyphenyl, o-, m-, p-Diethylaminoethoxyphenyl, 2-Thienyl, 3-Pyridyl, der Indol-3-yl-rest der Formel II, in dem $R^4$ Wasserstoff, Methyl, Ethyl, Benzyl, o-, m-, p-Chlorbenzyl oder Phenethyl bedeutet, in dem $R^5$ Wasserstoff, Methoxy oder Ethoxy bedeutet und in dem $R^6$ Wasserstoff oder Phenyl bedeutet.

Ein für $R^2$ stehender Alkanoylrest kann durch Chlor substituiert, vorzugsweise monosubstituiert sein. Der für $R^2$ stehende Phenylalkylrest kann im Phenylkern 1- oder 2-fach durch Chlor und bis zu 3-fach durch Alkoxy mit 1 oder 2 Kohlenstoffatomen substituiert sein. Bei Einfachsubstitution können die Substituenten in o-, m- oder p-Stellung zur Alkylenbrücke stehen, bei Zweifachsubstitution sind die Positionen 2,4-, 3,4-, 2,5- und 3,5- besonders zweckmäßig, und bei Dreifachsubstitution können die Substituenten vorzugsweise in 2,4,5-, 2,4,6- und 3,4,5-Stellung stehen. Eine für $R^2$ stehende Benzoylgruppe kann im Phenylrest durch 1 bis 3 Alkoxygruppen mit 1 oder 2 Kohlenstoffatomen oder durch 1 oder 2 Nitro- oder Aminogruppen substituiert sein. Für die Stellungen der Substituenten treffen die gleichen Angaben zu, wie für die für $R^2$ stehenden substituierten Phenalkylreste. Die Aminogruppe eines für $R^2$ stehenden Aminosulfonyls ist vorzugsweise N-substituiert und zwar durch 1 oder 2 Alkylgruppen mit je 1 bis 4, vorzugsweise 1 bis 2 C-Atomen, oder sie ist Glied eines Piperidin-, Piperazin-, 4-Alkylpiperazin- oder Morpholinrings, wobei das Alkylpiperazin vorzugsweise 1 bis 2 C-Atome in dem Alkylrest aufweist.

Ein für $R^7$ stehender 4-Alkylpiperazin-1-yl-Rest hat in der Alkylgruppe vorzugsweise 1 bis 2 C-Atome.

In einem für $R^8$ stehenden durch Chlor substituierten Phenyl können 1 oder 2 Chloratome enthalten sein, die entweder bei Monosubstitution in o-, m- oder p-Stellung zur NH-Gruppe, oder bei Disubstitution in 2,4-, 2,5-, 3,4- oder 3,5-Stellung stehen. In einem für $R^9$ stehenden Chlor-phenoxyacetylrest der Formel VI kann das Chloratom in o-, m- oder p-Position zum Sauerstoff stehen.

Beispiele für Substituenten, die für $R^2$ stehen können, sind Methyl, Ethyl, Propyl, Isopropyl, Butyl, Isobutyl, tert.-Butyl, Methoxycarbonylmethyl, Methoxycarbonylethyl, Ethoxycarbonylmethyl, Ethoxycarbonylethyl, Propoxycarbonylmethyl, Butoxycarbonylmethyl, Aminocarbonylmethyl, Aminocarbonylethyl, Dimethylaminocarbonylmethyl, Diethylaminocarbonylmethyl, Piperidinylcarbonylmethyl, Piperazinylcarbonylmethyl, Morpholinylcarbonylmethyl, Benzyl, o-, m-, p-Chlorbenzyl, 2,4-, 3,4-, 2,5-, 3,5-Dichlorbenzyl, o-, m-, p-Methoxybenzyl, o-, m-, p-Ethoxybenzyl, 2,4-, 2,5-, 3,4-, 3,5-Dimethoxybenzyl, 2,4-, 2,5-, 3,4-, 3,5-Diethoxybenzyl, 2,4,5-, 2,4,6-, 3,4,5-Trimethoxybenzyl, Phenethyl, o-, m-, p-Chlorphenethyl, 2,4-, 2,5-, 3,4- oder 3,5-Dichlorphenethyl, o-, m-, p-Methoxyphenethyl, o-, m-, p-Ethoxyphenethyl, 2,3-, 2,5-, 3,4- oder 3,5-Dimethoxyphenethyl, 2,4-, 2,5-, 3,4- oder 3,5-Diethoxyphenethyl, 2,4,5-, 2,4,6- oder 3,4,5-Trimethoxyphenethyl, 3-Phenylpropyl-(1), 3-(o-, m-, p-Methoxyphenyl)-propyl-(1), 3-(o-, m-, p-Ethoxyphenyl)-propyl-(1), Formyl, Acetyl, Chloracetyl, Propionyl, oder α-Chlorpropionyl, Butyryl, Nicotinoyl, Benzoyl, o-, m-, p-Methoxybenzoyl, o-, m-, p-Ethoxybenzoyl, 2,4-, 2,5-, 3,4- oder 3,5-Dimethoxybenzoyl, 2,4-, 2,5-, 3,4- oder 3,5-Diethoxybenzoyl, 2,4,5-, 2,4,6-oder 3,4,5-Trimethoxybenzoyl, o-, m-, p-Nitrobenzoyl, 2,4-Dinitrobenzoyl, o-, m-, p-Aminobenzoyl, 2,4-Diaminobenzoyl, Dimethylaminosulfonyl, Diethylaminosulfonyl, Aminoacetyl, Morpholinylacetyl, Aminocarbonyl, Phenylaminocarbonyl, o-, m-, p-Chlorphenylaminocarbonyl, Hydroxyethyl, Phenoxyacetoxyethyl, o-, m-, p-Chlorphenoxy-acetoxyethyl.

Besonders bevorzugte Bedeutungen für $R^2$ sind beispielsweise: Wasserstoff, Methyl, Ethyl, Aminocarbonylmethyl, Dimethylaminocarbonylmethyl, Methoxycarbonylmethyl, Ethoxycarbonylmethyl, Piperidinylcarbonylmethyl, Morpholinylcarbonylmethyl, Benzyl, o-, m-, p-Chlorbenzyl, o-, m-, p-Methoxybenzyl, o-, m-, p-Ethoxybenzyl, 2,4-, 2,5-, 3,4-, 3,5-Dimethoxybenzyl, Phenethyl, o-, m-, p-Methoxyphenethyl, o-, m-, p-Ethoxyphenethyl, 2,3-, 2,5-, 3,4- oder

3,5-Dimethoxyphenethyl, 3-Phenylpropyl-(1), Formyl, Acetyl, Chloracetyl, Propionyl, Nicotinoyl, Benzoyl, o-, m-, p-Methoxybenzoyl, 2,4-, 2,5-, 3,4- oder 3,5-Dimethoxybenzoyl, 3,4,5-Trimethoxybenzoyl, o-, m-, p-Nitrobenzoyl, o-, m-, p-Aminobenzoyl, Dimethylaminosulfonyl, Aminoacetyl, Morpholinylacetyl, Aminocarbonyl, Phenylaminocarbonyl, o-, m-, p-Chlorphenylaminocarbonyl, Hydroxyethyl, Phenoxyacetoxyethyl, o-, m-, p-Chlorphenoxyacetoxyethyl.

Eine an den Phenylrest einer für $R^3$ stehenden Gruppe der Formel VII gebundene Alkoxygruppe ($R^{10}$) hat vorzugsweise 1 bis 2 C-Atome und kann in o-, m-, p-Stellung zum Stickstoff des Piperazinringes stehen.

Beispiele für Substituenten, die für $R^3$ stehen können, sind: Methyl, Ethyl, Propyl, Isopropyl, Butyl-(1), Butyl-(2), Isobutyl, tert.-Butyl, Methoxycarbonylmethyl, Ethoxycarbonylmethyl, Propoxycarbonylmethyl, Butoxycarbonylmethyl, Aminocarbonylmethyl, Monomethylaminocarbonylmethyl, Monoethylaminocarbonylmethyl, Monopropylaminocarbonylmethyl, Dimethylaminocarbonylmethyl, Diethylaminocarbonylmethyl, 4-Phenylpiperazin-(1)-yl-carbonylmethyl, 4-(o-, m-, p-Methoxyphenyl)-piperazin-(1)-yl-carbonylmethyl, 4-(o-, m-, p-Ethoxyphenyl)piperazin-(1)-yl-carbonylmethyl.

Bevorzugte Bedeutungen für $R^3$ sind:

Wasserstoff, Methyl, Ethyl, Methoxycarbonylmethyl, Ethoxycarbonylmethyl, Propoxycarbonylmethyl, Butoxycarbonylmethyl, Aminocarbonylmethyl, Monomethylaminocarbonylmethyl, Monoethylaminocarbonylmethyl, Dimethylaminocarbonylmethyl, 4-Phenylpiperazin-(1)-yl-carbonylmethyl, 4-(o-, m-, p-Methoxyphenyl)-piperazin-(1) -yl-carbonylmethyl.

Besonders bevorzugt sind solche erfindungsgemäßen Piperazinone der Formel I, die mehrere der oben genannten bevorzugten Strukturmerkmale aufweisen.

Die Herstellung der erfindungsgemäßen Piperazinone der allgemeinen Formel I erfolgt dadurch, daß ein 5,6-Dihydro-2(1H)-pyrazin der Formel VIII

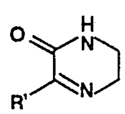

(VIII)

zu dem entsprechenden substituierten Piperazin-2-on der allgemeinen Formel I, wobei $R^2$ und $R^3$ Wasserstoff bedeuten, reduziert und gegebenenfalls danach mit einem Alkylierungs- bzw. Acylierungsmittel der Formel IX

$$X - R^2 \qquad (IX),$$

worin X ein anionisch abspaltbarer Rest ist, in an sich bekannter Weise umgesetzt und/oder gegebenenfalls das erhaltene Piperazin-2-on der Formel I, wobei $R^3$ Wasserstoff ist, mit einem Alkylierungsmittel der Formel X

$$X - R^3 \qquad (X)$$

worin X ein anionisch abspaltbarer Rest ist, in an sich bekannter Weise umgesetzt und das erhaltene substituierte Piperazin-2-on gewünschtenfalls in an sich bekannter Weise in ein physiologisch verträgliches Säureadditionssalz überführt wird.

Bevorzugte Piperazinone der Formel I werden erhalten, wenn man 5,6-Dihydro-2(1H)-pyrazinone der Formel VIII, Alkylierungsmittel bzw. Acylierungsmittel der Formel IX und Alkylierungsmittel der Formel X einsetzt, in denen $R^1$, $R^2$ bzw. $R^3$ die oben genannten besonders zweckmäßigen oder bevorzugten Bedeutungen haben.

Bei der Reduktion der 5,6-Dihydro-2(1H)-pyrazinone der Formel VIII zu entsprechenden erfindungsgemäßen Piperazinonen der Formel I, in denen $R^2$ und $R^3$ Wasserstoff sind, erfolgt eine Hydrierung der 3,4-Doppelbindung. Analoge Reduktionen sind in großer Zahl bekannt, und sowohl die geeigneten Reduktionsmittel, als auch die Reduktionsbedingungen können der Literatur entnommen werden. (Vgl. z. B. Methoden der Organ. Chemie (Houben-Weyl) 4. Auflage, Stuttgart 1957 Band 11/1, S. 692 ff.)

Besonders geeignet für die Herstellung der erfindungsgemäßen Piperazinone aus den Dihydropyrazinonen der Formel VIII sind katalytisch erregter Wasserstoff und komplexe Hydride, wie beispielsweise Alkali-Borhydrid oder Alkali-Aluminiumhydrid. Zweckmäßigerweise wird die Reduktion in einem inerten organischen Lösungsmittel durchgeführt. Bei Verwendung vom Wasserstoff als Reduktionsmittel können als Katalysatoren, Raney-Nickel oder Raney-Kobalt eingesetzt werden, wenn bei erhöhter Temperatur, vorzugsweise zwischen 50 und 100°C, gearbeitet wird oder Edelmetallkatalysatoren, wie feinverteiltes Platin oder Palladium, zweckmäßigerweise auf einem geeigneten Träger, wie z. B. Aktivkohle, wenn bei Raumtemperatur oder wenig erhöhter Temperatur gearbeitet wird. Die Reduktion mit komplexen Hydriden kann häufig bereits bei Zimmertemperatur ausgeführt werden, die Reaktion kann jedoch in üblicher Weise durch Erwärmen, z. B. bis zum Siedepunkt eines vorzugsweise unter 100°C siedenden Lösungsmittels, beschleunigt werden.

Als Lösungsmittel kommen bei Einsatz von katalytisch erregtem Wasserstoff z. B. niedere Alkohole, wie Methanol, Ethanol, Propanol; niedere Carbonsäuren wie Essigsäure; die Ester niederer Carbonsäuren mit einfachen Alkoholen, wie Essigsäureethylester und Äther, wie Ethylenglykolmono- oder dimethyläther oder Tetrahydrofuran in Betracht.

Die Reduktion mit Alkali-Borhydrid kann z. B. in einfachen Alkoholen, wie Methanol oder Ethanol, die mit

Alkali-Aluminiumhydrid, bevorzugt in wasserfreien Äthern wie Diäthyläther, Dioxan oder Tetrahydrofuran durchgeführt werden.

Aus den nach der Reduktion erhaltenen erfindungsgemäßen Piperazinonen der Formel I, in denen $R^2$ und $R^3$ Wasserstoff ist, lassen sich durch Alkylierung bzw. Acylierung bereits unter milden Bedingungen die Verbindungen der Formel I herstellen, in denen nur noch $R^3$ Wasserstoff ist, $R^2$ dagegen im Rahmen der oben angegebenen Definitionen eine von Wasserstoff verschiedene Bedeutung hat. Sowohl die für die Alkylierung bzw. Acylierung erforderlichen Alkylierungs- bzw. Acylierungsmittel als auch die Reaktionsbedingungen, unter denen Alkylierung- bzw. Acylierung ablaufen, sind aus der Literatur bekannt (Vgl. Methoden der organ. Chemie (Houben-Weyl) 4. Auflage, Stuttgart 1957, Band 11/1, S. 24 ff und 11/2, S. 3 ff). Im Prinzip werden stets Alkylierung- bzw. Acylierungsmittel der oben angegebenen Formel IX eingesetzt. Anionisch abspaltbare Reste X sind z.B. Halogenatome, insbesondere Chlor, Brom oder Jod, oder ein Äquivalent Sulfatogruppe oder, allerdings weniger üblich, eine quartäre Ammoniumgruppe oder eine ternäre Sulfoniumgruppe. Für den Fall, daß $R^2$ Alkyl oder substituiertes Alkyl ist, steht X vorzugsweise für Halogen wie Chlor, Brom oder Jod, und für den Fall, daß $R^2$ ein Acylrest ist, bedeutet X üblicherweise Halogen, insbesondere Chlor oder Brom, oder ein Rest der Formel $R^2O$.

Die Reaktion wird üblicherweise in einem inerten organischen Lösungsmittel durchgeführt. Zum Abfangen des abgespaltenen Protons wird zweckmäßigerweise eine Base dem Ansatz zugefügt. Sollen erfindungsgemäße Piperazinone der Formel I hergestellt werden, in denen auch die 1-Stellung substituiert ist, d.h. $R^3$ ungleich Wasserstoff ist, so werden die nach dem ersten Alkylierungs- bzw. Acylierungsschritt erhaltenen Verbindungen einen zweiten Alkylierungsschritt unterworfen.

Hierbei werden Alkylierungsmittel der Formel X - $R^3$ eingesetzt, in denen X im Prinzip die gleichen Bedeutungen hat, wie bereits oben für die Verbindungen der Formel X - $R^2$ angegeben. Auch diese zweite Alkylierung wird in der Regel in einem organischen Lösungsmittel durchgeführt und wird in Gegenwart einer Base gearbeitet. Die Reaktionsbedingungen werden hierbei gegenüber den Bedingungen der ersten Alkylierung verschärft, d.h. es wird in der Regel in einem stärker polaren Lösungsmittel und mit einer aktiveren, z. B. einer in dem Reaktionsmedium löslichen Base gearbeitet. Als Lösungsmittel für die Umsetzung mit Verbindungen der Formel X - $R^2$ können beispielsweise niedere Alkohole, d.h. solche mit 1 - 4 C-Atomen, Benzolderivate, wie Toluol, Chlorbenzol, Äther und cyclische Äther, wie beispielsweise Tetrahydrofuran oder Dioxan eingesetzt werden. Als Lösungsmittel für die Umsetzung mit Verbindung der Formel X - $R^3$ werden polare Lösungsmittel, wie beispielsweise Dimethylformamid, Dimethylsulfoxid oder Pyridin, bevorzugt.

Als Basen für den ersten Alkylierungs- bzw. Acylierungsschritt mit Verbindungen der Formel X - $R^2$ genügen anorganische Verbindungen, die in dem verwendeten Medium nicht löslich sein müssen, wie z. B. Alkalisalze schwacher Säuren, z. B. Alkaliacetate, Alkalicarbonnate, Alkalihydrogencarbonate, oder Alkaliphosphate, aber auch Magnesiumoxyd, oder Kalziumoxyd. Die bei der Alkylierung mit Verbindungen der Formel X - $R^3$ eingesetzten Basen sind vorzugsweise in dem Reaktionsmedium löslich, zweckmäßigerweise werden in diesem Fall Alkalihydroxide oder Alkalialkoholate eingesetzt.

Zur Herstellung von Säureadditionssalzen der erfindungsgemäßen Verbindungen der Formel I werden letztere in einem organischen Lösungsmittel gelöst, und mit einer Lösung der gewünschten Säure in einem zweckmäßigerweise mit dem als Lösungsmittel für I eingesetzten mischbaren, organischen Lösungsmittel versetzt. So können beispielsweise die Hydrochloride der erfindungsgemäßen Piperazinone der Formel I dadurch erhalten werde, daß die Verbindungen in Alkohol gelöst werden und die alkoholische Lösung mit einer äquivalente Menge einer Lösung von Chlorwasserstoff in Äther versetzt werden.

Die erfindungsgemäßen Piperazinone der Formel I und ihre physiologisch verträglichen Salze sind Nootropika, d.h. sie dienen zur Behandlung von Krankheiten, die durch eine Einschränkung der Gehirnfunktion, insbesondere der Gedächtnisleistung, charakterisiert sind, sowie zur Minderung zerebraler Alterungsvorgänge. Sie sind den bisher bekannten Verbindungen gleicher Wirkungsrichtung überraschenderweise erheblich überlegen. Sie zeigen eine ausgezeichnete Wirksamkeit in verschieden artigen Tests, wie z. B. bei der Verlängerung der Überlebenszeit unter Natriumnitrit-Hypoxie nach Gibsen und Bless (J. Neurochemistry *27* (1976)) bei der Verbesserung der stickstoffinduzierten Hypoxietoleranz, wobei Versuchstiere nach Prämedikation mit dem untersuchten Präparat mit reinem Stickstoff beatmet werden und die Verlängerung des Zeitraums zwischen Einsatz der Beatmung und elektrischer Neutralität des Elektroencephalogramms sowie die Letalität gemessen wird. Auch in Tests, die direkt auf die Erfassung der Lern- und Gedächtnisleistung abzielen, wie z. B. den bekannten "avoidance"-Tests sind die erfindungsgemäßen Produkte sehr gut wirksam.

Die Prüfung in den genannten und einer Reihe weiterer Tests ergibt, daß die erfindungsgemäßen Verbindungen bei geringer Toxizität überraschenderweise ein besonders günstiges, bei bekannten Präparaten in dieser Form nicht vorliegendes Wirkungsprofil aufweisen.

Die erfindungsgemäßen Piperazinone können daher am Menschen für sich allein, in Mischungen untereinander oder in pharmazeutischen Zubereitungen verabreicht werden, die als aktiven Bestandteil eine wirksame Dosis mindestens eines erfindungsgemäßen Piperazinons oder eines Säureadditionssalzes davon neben üblichen pharmazeutisch einwandfreien Träger- und Zusatzstoffen enthalten.

Geeignete Trägerstoffe sind z. B. Wasser, pflanzliche Öle, Stärke, Gelatine, Milchzucker, Magnesiumstearat, Wachse, Vaseline, etc. Als Zusatzstoffe können z. B. Netzmittel, Sprengmittel, Konservierungsmittel etc. verwendet werden.

Die geeigneten Dosen der Wirkstoffe können in weiten Grenzen variiert und so an die individuellen Erfordernisse des Einzelfalls angepaßt werden. In der Regel beträgt die Tagesdosis 0,1 bis 150 mg, vorzugsweise 1 bis 30 mg pro Patient bei oraler Application. Auch bei anderweitiger Application liegen die Tagesdosen dank der

guten Resorbierbarkeit der Wirkstoffe in den gleichen Mengenbereich.

Die Tagesdosis kann auf einmal gegeben werden, wird jedoch in der Regel in mehreren, beispielsweise 2 bis 4, Teildosen verabreicht. Die Einzeldosis der wirksamen Substanz beträgt dabei in der Regel 0,001 bis 1 mg pro kg Körpergewicht. Pharmazeutische Präparate enthalten normalerweise 0,1 bis 50 mg, vorzugsweise 0,5 bis 10 mg Wirkstoff der Formel I oder eines pharmakologisch annehmbaren Salzes pro Dosis.

Die pharmazeutischen Präparate können in Form von z. B. Tabletten, Kapseln, wäßrigen oder öligen Lösungen oder Suspensionen, Emulsionen, injizierbaren wäßrigen oder öligen Lösungen oder Suspensionen, dispergierbaren Pulvern oder Aerosolmischungen vorliegen. Die pharmazeutischen Präparate können neben den Verbindungen der allgemeinen Formel I auch noch eine oder mehrere andere pharmazeutisch wirksame Substanzen, beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen wie Digoxin, Acetyldigoxin, Metildigoxin und Lanato-Glykoside; Coronardilatatoren, wie Carbocromen, Dipyridamol, Nifedipin und Perhexilin; antianginöse Verbindungen wie Isosorbiddinitrat, Isosorbidmononitrat, Glyceroltrinitrat, Molsidomin und Verapamil; β-Blocker wie Propranolol, Oxprenolol, Atenolol, Metoprolol und Penbutolol und oogenmetabolische Mittel, wie Pirilinol. Darüber hinaus lassen sich die Verbindungen mit anderen nootrop wirkenden Substanzen, wie z. B. Piracetam kombinieren.

Die folgenden Ausführungsbeispiele veranschaulichen die Herstellung der erfindungsgemäßen Verbindungen. Die Reaktionsbedingungen die Reduktions-, Alkylierungs- und Acylierungsmittel können im Rahmen des Standes der Technik und der Patentansprüche und die eingesetzten 5,6-Dihydro-2(1H)-pyrazinone im Rahmen der Patentansprüche variiert werden.

**Beispiel 1:**

**3-(3,4-Dimethoxyphenyl)-piperazin-2-on:**

23,4 g (0,1 Mol) 3-(3,4-Dimethoxyphenyl-)-5,6-dihydro-2(1 H)-pyrazinon werden in 230 ml Ethanol gelöst und in Gegenwart von Raney-Nickel als Katalysator bei 40 bis 60°C und 50 bar Wasserstoffdruck bis zur Sättigung hydriert. Danach wird vom Katalysator abfiltriert, das Filtrat im Vakuum eingedampft und der Rückstand mit Äther verrieben.

Ausbeute: 22,9 g = 97,8 % d. Theorie (Molgewicht 236),
Schmelzpunkt: 124 - 126°C.

**Beispiel 2:**

**3-(3,4-Dimethoxyphenyl)-4-benzyl-piperazin-2-on:**

47,2 g (0,2 Mol) des nach Beispiel 1 hergestellten 3-(3,4-Dimethoxyphenyl)-piperazin-2-on werden in 500 ml Chlorbenzol zusammen mit 27,8 g (0,22 Mol) Benzylchlorid und 23,3 g (0,22 Mol) Natriumcarbonat 12 Stunden rückfliessend gekocht.

Nach dem Abkühlen saugt man die Reaktionsmischung ab, dampft das Filtrat im Vakuum ein, nimmt den Rückstand in Methylenchlorid auf, schüttelt die Lösung gründlich mit Wasser aus, dampft das Methylenchlorid ab. Den Rückstand verrührt man mit etwas Äther, saugt erneut ab und trocknet den Filterrückstand.

Ausbeute: 44 g ≈ 67,5 % d. Theorie (Molgewicht 326),
Schmelzpunkt: 167 - 168°C.

Durch Lösen der Substanz in wenig Alkohol und Hinzufügen von mit Chlorwasserstoff gesättigtem Äther erhält man das Chlorhydrat vom Schmelzpunkt 231 - 234°C.

**Beispiel 3:**

**3,(3,4-Dimethoxyphenyl)-4-acetyl-piperazin-2-on**

23,6 g (0,1 Mol) des nach Beispiel 1 hergestellten 3-(3,4-Dimethoxyphenyl-)-piperazin-2-on werden in 100 ml Acetanhydrid eingetragen.

Nach Abklingen der exothermen Reaktion wird noch 2 Stunden bei Raumtemperatur nachgerührt, im Vakuum eingeengt, der Rückstand zuerst mit wässriger Ammoniaklösung, dann mit Wasser gewaschen und in Methylenchlorid aufgenommen. Nach dem Trocknen der Lösung wird sie im Vakuum eingedampft, der Rückstand mit wenig Äther verrieben und abgesaugt.

Ausbeute: 20,1 g = 72,3 % d. Theorie (Molgewicht 278),
Schmelzpunkt: 169 - 170°C.

**Beispiel 4:**

**3-(3,4-Dimethoxyphenyl-)-4-carbonamidopiperazin-2-on:**

23,6 g (0,1 Mol) des nach Beispiel 1 hergestellten 3-(3,4-Dimethoxyphenyl)-piperazin-2-on werden mit 250 ml Wasser und 10 ml (0,1 Mol) 10 n Salzsäure verrührt und 8,1 g (0,1 Mol) Kaliumcyanat, in 20 ml Wasser gelöst, zugetropft. Man rührt 8 Stunden bei Raumtemperatur und saugt ab. Der Filterrückstand wird mit Wasser gewaschen und mit 150 ml Ethanol aufgekocht, wieder abgekühlt, erneut abgesaugt und getrocknet.

Ausbeute: 20 g = 78,7 % d. Theorie (Molgewicht 254),
Schmelzpunkt: 196 - 199°C.

**Beispiel 5:**

**3-(3,4-Dimethoxyphenyl)-4-formyl-piperazin-2-on:**

23,6 g (0,1 Mol) 3-(3,4-Dimetoxyphenyl)-piperazin-2-on, hergestellt nach Beispiel 1, werden mit 150 ml Ameisensäuremethylester in einem Autoklaven 3 Stunden auf 110°C erhitzt. Der nach dem Einengen im Vakuum verbleibende Rückstand wird mit Äther verrieben, abgesaugt und getrocknet.

Ausbeute: 24 g = 91 % d. Theorie (Molgewicht 264),
Schmelzpunkt: 125 - 127°C.

**Beispiel 6:**

**3-(3,4-Dimethoxyphenyl)-4-carbonamidomethyl-piperazin-2-on:**

10 g (0,03 Mol) 3-(3,4-Dimethoxyphenyl)-4-carbomethoxymethyl-piperazin-2-on, 180 ml Ethanol und 20 ml flüssiger Ammoniak werden in einem Autoklaven 12 Stunden auf 70 - 80°C erhitzt. Nach dem Erkalten wird das Produkt abgesaugt, mit Ethanol gewaschen und getrocknet.

Ausbeute: 5,3 g = 60 % d. Theorie (Molgewicht 293),
Schmelzpunkt: 230 - 231°C.

Das Ausgangsprodukt wird wie folgt hergestellt: 23,6 g (0,1 Mol) 3-(3,4-Dimethoxyphenyl)-piperazin-2-on (Beispiel 1) und 12 g Chloressigsäuremethylester werden in 150 ml Chlorbenzol in Gegenwart von 11,7 g (0,11 Mol) Natriumcarbonat 12 Stunden rückfliessend gekocht. Dann wird das Reaktionsgemisch eingedampft, in Wasser/Methylenchlorid aufgenommen, die organische Schicht abgetrennt, eingedampft, der verbleibende Rückstand mit Äther verrieben und abgesaugt.

Ausbeute: 19,7 g ≈ 64 % d. Theorie (Molgewicht 308),
Schmelzpunkt: 116 - 118°C.

**Beispiel 7:**

**3-(3,4-Dimethoxyphenyl)-4-aminoacetyl-piperazin-2-on-hydrochlorid:**

7,5 g (0,024 Mol) 3-(3,4-Dimethoxyphenyl-)-4-chloracetyl-piperazin-2-on, 400 ml Methanol und 20 ml flüssiges Ammoniak werden 12 Stunden im Autoklaven auf 80 - 90°C erhitzt. Nach dem Abkühlen wird das Reaktionsgemisch im Vakuum eingedampft, der Rückstand in wenig Methanol gelöst, filtriert und wieder eingedampft.

Ausbeute: 5,8 g = 73 d. Theorie (Molgewicht 329),
Schmelzpunkt: 140°C Zersetzung.

Das Ausgangsprodukt wird wie folgt hergestellt:
23,6 g (0,1 Mol) 3-(3,4-Dimethoxyphenyl)-piperazin-2-on (Beispiel 1) 150 ml Chloroform und 10,1 g Triethylamin werden unter Kühlung mit einer Lösung von 11,5 g (0,1 Mol) Chloracetylchlorid in 50 ml Chloroform versetzt. Man rührt 1 Stunde bei Raumtemperatur nach, engt im Vakuum ein, nimmt den Rückstand in wässriger Kaliumcarbonatlösung auf und schüttelt die Lösung mit Essigsäureethylester aus. Nach dem Abdestillieren des Essigsäureethylesters erhält man 17,5 g ≈ 56 % d. Theorie 3-(3,4-Dimethoxyphenyl)-4-chloracetyl-piperazin-2-on.

Molgewicht 312,5, Schmelzpunkt: 102 -106°C.

**Beispiel 8:**

**3-(3-4-Dimethoxyphenyl)-4-morpholinoacetyl-piperazin-2-on:**

7,8 g des nach Beispiel 7 hergestellten 3-(3,4-Dimethoxyphenyl)-4-chloracetyl-piperazin-2-on, 50 ml Toluol und 4,5 g (0,05 Mol) Morpholin werden 3 Stunden rückfliessend gekocht. Danach wird das Reaktionsgemisch im Vakuum eingeengt, der Rückstand mit einer wässrigen Lösung von Kaliumcarbonat versetzt und mit Methylenchlorid ausgeschüttelt. Die organische Phase wird abgetrennt und Methylenchlorid abgedampft.

Ausbeute: 6 g ≈ 66 % d. Theorie (Molgewicht 363),
Schmelzpunkt: 136 - 137°C.

**Beispiel 9:**

**1-Methyl-3-(3,4-dimethoxyphenyl)-4-benzyl-piperazin-2-on:**

32,6 g (0,1 Mol) 3-(3,4-Dimethoxyphenyl)-4-benzyl-piperazin-2-on, hergestellt nach Beispiel 2, werden in 150 ml Dimethylsulfoxyd gelöst, mit 12,3 g (0,11 Mol) Kalium-tert.butylat 15 Minuten bei Raumtemperatur gerührt und unter Kühlung mit 15,6 g (0,11 Mol) Methyljodid versetzt. Nach 1 Stunde wird das Dimethylsulfoxyd im Vakuum abdestilliert, der Rückstand mit Wasser versetzt, alkalisch gestellt und mit Essigester ausgeschüttelt. Nach dein Abtrennen der organischen Phase und dem Abdestillieren des Lösungsmittels bleibt die Verbindung als zähflüssiges Öl zurück.

Ausbeute: 35,4 g. Durch Lösen in Isopropanol und Versetzen mit einer äquivalenten Menge Naphthalin-1,5-disulfonsäure erhält man das Naphthalin-disulfonat vom Schmelzpunkt 162 - 165°C.

**Beispiel 10:**

**1-Carbonamidomethyl-3-(3,4-dimethoxyphenyl)-4-benzyl-piperazin-2-on:**

17,5 g (0,044 Mol) 1-Carbomethoxymethyl-3-(3,4-dimethoxyphenyl)-4-benzylpiperazin-2-on werden mit 150 ml Ethanol und 50 ml flüssigem Ammoniak im Autoklaven 12 Stunden auf 70 - 80°C erhitzt. Nach dem Einengen des Reaktionsproduktes im Vakuum wird der Rückstand in Essigester aufgenommen und das Lösungsmittel abdestilliert. Durch Verreiben des Rückstandes mit Äther und Absaugen erhält man 9,5 Rohprodukt, das aus 70 ml Ethanol umkristallisiert wird.

Ausbeute: 6 g ≈ 36 % d. Theorie (Molgewicht 382),
Schmelzpunkt: 206 - 208°C.

Das als Ausgangsprodukt dienende 1-Carbomethoxymethyl-3-(3,4-dimethoxyphenyl)-4-benzyl-piperazin-2-on wird folgendermassen hergestellt: 40 g (0,12 Mol) 3-(3,4-Dimethoxyphenyl)-4-benzyl-piperazin-2-on, hergestellt gemäß Beispiel 2, werden in eine Lösung von 20 g (0,18 Mol) Kalium-tert.butylat in 250 ml Dimethylsulfoxid eingetragen. Es wird 15 Minuten bei Zimmertemperatur gerührt, 19,5 (0,18 Mol) Chloressigsäuremethylester eingetropft und 6 Stunden bei 40 - 50°C gerührt. Nach dem Abdestillieren des Dimethylsulfoxides im Vakuum, wird der Rückstand mit einer Lösung von Kaliumcarbonat in Wasser versetzt und mit Essigester ausgeschüttelt. Nach Abtrennung der organischen Phase und dem Abdestillieren des Essigesters bleiben 43,5g eines zähflüssigen Öls zurück, das ohne weitere Reinigung verwendet wird.

**Beispiel 11:**

**1-Carbonamidomethyl-3-(3,4-dimethoxyphenyl)-piperazin-2-on-Tartrat:**

6,5 g (0,02 Mol)1-Carbonamidomethyl-3-(3,4-dimethoxyphenyl)-4-benzyl-piperazin-2-on, hergestellt gemäß Beispiel 10, werden in 80 ml Eisessig gelöst und bei Raumtemperatur in Gegenwart eines Palladiumkohle-Katalysators bis zur Sättigung hydriert. Danach wird das Reaktionsgeimisch vom Katalysator befreit, im Vakuum eingeengt, der Rückstand mit wässriger Kaliumcarbonatlösung alkalisch gestellt, das ausfallende Öl in Dimethoxyethan gelöst und das 1-Carbonamidomethyl-3-(3,4-dimethoxyphenyl)-piperazin-2-on durch Zusatz von Weinsäure als Tartrat gefällt.

Ausbeute: 3,4 g ≈ 38 % d. Theorie (Molgewicht 443).

**Beispiel 12:**

**1-[ 4-( 2-Methoxyphenyl )-piperazin-1-carbomethyl ]-3-( 3,4-dimethoxyphenyl )-4-benzyl-piperazin-2-on-Sulfat:**

17,1 g (0,043 Mol) 1-Carbomethoxymethyl-3-(3,4-dimethoxyphenyl)-4-benzyl-piperazin-2-on, hergestellt gemäß Beispiel 10, 100 ml Glykolmono-methylether und 9,1 g (0,047 Mol) 1-(2-Methoxyphenyl)-piperazin werden 10 Stunden rückfiessend gekocht. Nach dem Einengen des Reaktionsgemisches im Vakuum wird es mit Wasser und Essigester verrührt, die Essigesterphase abgetrennt, getrocknet, eingeengt und das zurückbleibende Öl in wenig Ethanol gelöst. Durch Zugabe einer Lösung von 1,7 g Schwefelsäure in Ethanol fällt das obengenannte Sulfat. Es wird abgesaugt und mit Äther gewaschen.

Ausbeute: 6,6 g ≈ 23 % d. Theorie (Molgewicht 654),
Schmelzpunkt: 215 - 217°C.

**Beispiel 13:**

**3-(4-Fluorphenyl)-piperazin-2-on:**

24 g (0,125 Mol) 3-(4-Fluorphenyl)-5,6-dihydro-2(1 H)pyrazinon werden, wie in Beispiel 1 beschrieben, hydriert, aufgearbeitet und aus Essigsäureethylester umkristallisiert.
Ausbeute: 15 g = 61,8 % d. Theorie (Molgewicht 194),
Schmelzpunkt: 105 - 108°C.

**Beispiel 14:**

**3-(4-Fluorphenyl)-4-benzyl-piperazin-2-on:**

25 g (0,129 Mol) 3-(4-Fluorphenyl)-piperazin-2-on, hergestellt gemäß Beispiel 13, werden, wie in Beispiel 2 beschrieben, mit 18,1 g (0,143 Mol) Benzylchlorid alkyliert. Ausbeute: 24,3 g = 66,3 % d. Theorie (Molgewicht 284),
Schmelzpunkt: 170 - 172°C.

**Beispiel 15:**

**3-(4-Fluorphenyl)-4-formyl-piperazin-2-on:**

10 g (0,051 Mol) 3-(4-Fluorphenyl)-piperazin-2-on, hergestellt gemäß Beispiel 13, werden, wie in Beispiel 5 beschrieben, durch Erhitzen mit Ameisensäuremethylester acyliert.
Ausbeute: 6 g = 53 % (Molgewicht 222),
Schmelzpunkt: 120 - 123°C.
Verwendet man als Acylierungskomponente Acetanhydrid und arbeitet analog Beispiel 3, so erhält man das

**3-(4-Fluorphenyl)-4-acetyl-piperazin-2-on**

in 57 % Ausbeute, Molgewicht 236, Schmelzpunkt 139 - 140°C.

**Beispiel 16:**

**3-(2-Thienyl)-piperazin-2-on:**

22,2 g (0,123 Mol) 3-(2-Thienyl)-5,6-dihydro-2-(1 H)-pyrazinon werden, wie in Beispiel 1 beschrieben, hydriert und aufgearbeitet.
Ausbeute: 17 g 3-(2-Thienyl)-piperazin-2-on ≈ 75,9 % d. Theorie (Molgewicht 182),
Schmelzpunkt: 118°C.

**Beispiel 17:**

**3-(2-Thienyl)-4-formyl-piperazin-2-on:**

9,1 g (0,05 Mol) 3-(2-Thienyl)-piperazin-2-on (Beispiel 16) werden, wie in Beispiel 5 beschrieben, mit Ameisensäuremethylester umgesetzt.
Ausbeute: 8 g 3-(2-Thienyl)-4-formyl-piperazin-2-on 76 % d. Theorie (Molgewicht 210)
Schmelzpunkt: 127 - 129°C.
In Analogie zu Beispiel 3 wurden durch die Verwendung der entsprechenden Acylierungsmittel folgende Verbindungen hergestellt:
Unter Einsatz von Acetanhydrid als Acylierungsmittel

**3-(2-Thienyl)-4-acetyl-piperazin-2-on,**

Ausbeute: 81 % d. Theorie,
Schmelzpunkt: 144 - 146°C.
Unter Einsatz von Nicotinsäureanhydrid

**3-(2-Thienyl)-4-nicotinoyl-piperazin-2-on,**

Ausbeute: 71 % d. Theorie,
Schmelzpunkt: 240 - 241°C (Hydrochlorid).
Unter Einsatz von Trimethoxybenzoylchlorid

**3-(2-Thienyl-)-4-(3,4,5-trimethoxybenzoyl)-piperazin-2-on,**

Ausbeute: 63 % d. Theorie,
Schmelzpunkt: 164 - 165°C.
Unter Einsatz von 4-Nitrobenzoylchlorid

**3-(-2-Thienyl-)-4-(-4-nitrobenzoyl-)-piperazin-2-on**

Ausbeute: 83 % d. Theorie,
Schmelzpunkt: 204°C.
Durch katalytische Reduktion der Nitrogruppe, erhält man unter Anwendung der in Beispiel 1 beschriebenen Bedingungen das

**3-(2-Thienyl)-4-(4-aminobenzoyl-)piperazin-2-on**

Ausbeute: 70 d. Theorie,
Schmelzpunkt: 181 - 182°C.

**Beispiel 18:**

**1-Carboethoxymethyl-3-(2-thienyl-)-4-acetyl-piperazin-2-on:**

12 g (0,053 Mol) 3-(2-Thienyl)-4-acetyl-piperazin-2-on, hergestellt gemäß Beispiel 17, werden mit 6,6 g (0,059 Mol) Kalium-tert.butylat in 100 ml Dimethylsulfoxyd gerührt. Nach dem Abdestillieren von 10 ml Dimethylsulfoxid im Vakuum tropft man 6,4 g (0,059 Mol) Chloressigsäureethylester bei Raumtemperatur zu und rührt 12 Stunden nach. Das Dimethylsulfoxid wird im Vakuum abbedampft, der Rückstand mit Wasser verrührt, in Essigester aufgenommen und die organische Phase abgetrennt. Nach dem Abdestillieren des Essigesters erhält man 13,8 g des obengenannten Produktes 88 % d. Theorie (Molgewicht 296) in Form eines zähflüssigen Öls.

**Beispiel 19:**

**1-Carbonamidomethyl-3-(2-thienyl)-4-acetyl-piperazin-2-on:**

13,9 g (0,047 Mol) 1-Carbomethoxymethyl-3-(2-thienyl-)-4-acetyl-piperazin-2-on, hergestellt gemäß Beispiel 18, werden in 180 ml Ethanol gelöst und mit 20 ml flüssigem Ammoniak im Autoklaven 12 Stunden auf 80 - 90°C erhitzt. Das Reaktionsgemisch wird im Vakuum eingedampft, der Rückstand mit Methanol und Aktivkohle ausgekocht, die methanolische Lösung filtriert und eingedampft.
Ausbeute: 6 g 45,4 % d. Theorie. (Molgewicht 281),
Schmelzpunkt: 162 - 165°C.

**Beispiel 20:**

**3-(2-Thienyl-)-4-carbonamido-piperazin-2-on:**

9,1 g (0,05 Mol) 3-(2-Thienyl)-piperazin-2-on, hergestellt gemäß Beispiel 16, werden, wie in Beispiel 4 beschrieben, mit Kaliumcyanat umgesetzt und aufgearbeitet.
Ausbeute: 8,8 g 78 % d. Theorie, (Molgewicht 225),
Schmelzpunkt: 219 - 222°C.

**Beispiel 21:**

**3-(2Thienyl-)-4-chloracetyl-piperazin-2-on:**

18,2 (0,1 Mol) 3-(2-Thienyl-)piperazin-2-on, hergestellt gemäß Beispiel 16, werden in 150 ml Chloroform gelöst und mit 11,1 (0,11 ) Mol) Triäthylamin und 12,4 g (0,11 Mol) Choracetylchlorid versetzt. Man rührt 4 Stunden bei Raumtemperatur, engt die Reaktionslösung im Vakuum ein und verrührt den Rückstand mit Wasser und Essigester. Die Essigesterlösung wird abgetrennt, getrocknet und im Vakuum eingedampft.

Ausbeute: 19,7 g 76 % d. Theorie (Molgewicht 259) zähflüssiges Öl.

**Beispiel 22:**

Durch die Umsetzung der nach Beispiel 21 erhaltenen Verbindung mit Ammoniak bzw. Morpholin, wie in Beispiel 7 beschrieben, erhält man

**3-(2-Thienyl-)-4-aminoacetyl-piperazin-2-on:**

Schmelzpunkt: 130°C (Zersetzung). bzw.

**3-(2-Thienyl-)-4-(morpholinomethylcarbonyl)-piperazin-2-on:**

Schmelzpunkt: 144 - 146°C.

**Beispiel 23:**

**3-2-Thienyl-)-4-(3-chlorphenylaminocarbonyl-)-piperazin-2-on:**

5,5 g (0,03 Mol) 3-(2-Thienyl-)-piperazin-2-on, hergestellt gemäß Beispiel 16, werden in 60 ml Methylenchlorid gelöst, 4,6 g (0,03 Mol) 3-Chlorphenylisocyanat, gelöst in 20 ml Methylenchlorid, zugetropft und 5 Stunden bei Raumtemperatur nachgerührt. Das ausgefallene Reaktionsprodukt wird abgesaugt, mit Methylenchlorid gewaschen und getrocknet.

Ausbeute: 8,2 g 81 % d. Theorie (Molgewicht 335,5), Schmelzpunkt: 173 - 175°C.

**Beispiel 24:**

**3-(2-Thienyl-)-4-carbomethoxymethyl-piperazin-2-on:**

18,2 g (0,1 Mol) 3-(2-Thienyl-)piperazin-2-on, hergestellt gemäß Beispiel 16, werden, wie in Beispiel 6 beschrieben, mit Chloressigsäuremethylester umgesetzt und aufgearbeitet.

Ausbeute: 18,6 g 73 % d. Theorie (Molgewicht 254),
Schmelzpunkt: 119 - 123°C.

**Beispiel 25:**

**3-(2-Thienyl-)-4-carbonamidomethyl-piperazin-2-on:**

8,9 g (0,035 Mol) des gemäß Beispiel 24 hergestellten 3-(2-Thienyl)-4-carbomethoxymethyl-piperazin-2-on werden, wie in Beispiel 6 beschrieben, mit Ammoniak umgesetzt.

Ausbeute: 4,7 g = 56 % d. Theorie (Molgewicht 239),
Schmelzpunkt: 215 - 217°C.

Verwendet man als Reaktionspartner an Stelle von Ammoniak Morpholin, so erhält man

**3-(2-Thienyl)-4-morpholincarbonylmethyl-piperazin-2-on,**

Schmelzpunkt: 190 - 193°C.

**Beispiel 26:**

**3-(2-Thienyl-)-4-dimethylaminosulfonyl-piperazin-2-on:**

9,1 g (0,05 Mol) 3-(2-Thienyl)-piperazin-2-on, hergestellt gemäß Beispiel 16, werden mit einer Lösung von 6,1 g (0,06 Mol) Triäthylamin, in 100 ml Chloroform gemischt und 8,6 g (0,06 Mol) Dimethylaminosulfonsäurechlorid langsam zugetropft. Dann wird 6 Stunden bei Raumtemperatur nachgerührt, das Reaktionsgemisch im Vakuum eingedampft, der Rückstand mit Wasser verrührt und mit Essigester ausgeschüttelt. Die Essigester-Phase wird abgetrennt und nach dem Abdestillieren des Essigesters der Rückstand aus Ethanol umkristallisiert.

Ausbeute; 0,7 g ≈ 67 % d. Theorie (Molgewicht 289),
Schmelzpunkt: 134 - 136°C.

**Beispiel 27:**

**3-(2-Thienyl)-4-hydroxyethyl-piperazin-2-on-Tartrat:**

18,2 g (0,1 Mol) 3-(2-Thienyl)-piperazin-2-on, hergestellt gemäß Beispiel 16, werden in 180 ml Ethanol mit 13,2 g (0,3 Mol) Ethylenoxid im Autoklaven 24 Stunden bei 60°C gerührt. Das Reaktionsgemisch wird im Vakuum eingeengt, der Rückstand in Essigester gelöst und mit einer heißen Lösung von Weinsäure in Essigester versetzt. Das ausgefallene Tartrat wird abgesaugt, mit Essigester gewaschen und getrocknet.

Ausbeute: 28 g ≈ 74 % d. Theorie (Molgewicht 376),
Schmelzpunkt: 60 - 63°C.

**Beispiel 28:**

**3-(2-Thienyl)-4-(4'-chlorophenoxymethylcarbonyloxy-ethyl)-piperazin-2-on-Hydroxid:**

18,1 g (0,08 Mol) 3-(2-Thienyl)-4-hydroxyethyl-piperazin-2-on, hergestellt gemäß Beispiel 27, werden in 160 ml Chloroform gelöst und mit 10,1 g (0,1 Mol) Triethylamin und 20,5 g (0,1 Mol) 4-Chlorphenoxyacetylchlorid acyliert. Es wird 4 Stunden bei Raumtemperatur nachgerührt, eingedampft, der Rückstand mit Essigester ausgeschüttelt. Nach dem Trocknen des Essigesterauszuges mit Natriumsulfat wird die Verbindung durch Zugabe ätherischer Salzsäure als Hydrochlorid gefällt.

Ausbeute: 28 g ≈ 65 % d. Theorie (Molgewicht 431),
Schmelzpunkt: 211 - 213°C.

**Beispiel 29:**

**3-(2-Thienyl-)-4-benzyl-piperazin-2-on:**

54,6 g (0,3 Mol) 3-(2-Thienyl)-piperazin-2-on, hergestellt gemäß Beispiel 16, werden, wie in Beispiel 2 beschrieben, mit 41,7 g (0,33 Mol) Benzylchlorid umgesetzt.

Ausbeute: 61,7 g ≈ 75,6 % d. Theorie (Molgewicht 272),
Schmelzpunkt: 161°C (Zersetzung).

Unter Verwendung der entsprechend substituierten Benzylchloride wurden in analoger Weise folgende Verbindungen erhalten:

**3-(2-Thienyl)-4-(4-chlorbenzyl)-piperazin-2-on,**

Ausbeute: 78 % d. Theorie,
Schmelzpunkt: 191 - 193°C.

**3-(2-Thienyl-)-4-(2-chlorbenzyl)-piperazin-2-on,**

Ausbeute: 70 % d. Theorie,
Schmelzpunkt: 156°C.

**3-(2-Thienyl-)4-(3,4-dichlorbenzyl)-piperazin-2-on**

Ausbeute: 80 % d. Theorie,
Schmelzpunkt: 127°C.

**3-(2-Thienyl-)-4-(4-methoxybenzyl)-piperazin-2-on,**

Ausbeute: 80 % d. Theorie,
Schmelzpunkt: 216 - 218°C.
Verwendet man analog 3,4-Dimethoxyphenethylbromid als Alkylierungsmittel, so erhält man in einer Ausbeute von 68 % d. Theorie

**3-(2-Thienyl)-4-(3,4-dimethoxyphenethyl)-piperazin-2-on**

vom Schmelzpunkt: 261 - 262° (Naphthalin-1,5-disulfonat)

**Beispiel 30:**

**3-(3-Indolyl-)-piperazin-2-on:**

21,3 g (0,1 Mol) 3-(3-Indolyl)-5,6-dihydro-2-(1 H)-pyrazinon werden, wie in Beispiel 1 beschrieben, hydriert.
Ausbeute: 18 g 84 % d. Theorie (Molgewicht 215),
Schmelzpunkt: 152 - 155°C.

**Beispiel 31:**

**3-(3-Indolyl-)-4-formyl-piperazin-2-on:**

21,5 g (0,1 Mol) 3-(3-Indolyl-)-piperazin-2-on (Beispiel 30) werden, wie in Beispiel 5 beschrieben, mit Ameisensäuremethylester formyliert.
Ausbeute: 19,5 g = 80 % d. Theorie (Molgewicht 243)
Schmelzpunkt: 260 - 262°C.

**Beispiel 32:**

**3-(3-Indolyl-)-4-benzyl-piperazin-2-on:**

21,5 g (0,1 Mol) 3-(3-Indolyl-)-piperazin-2-on, hergestellt gemäß Beispiel 30, werden wie in Beispiel 2 beschrieben, mit Benzylchlorid alkyliert.
Ausbeute: 27 g ≈ 88 % d Theorie (Molgewicht 305),
Schmelzpunkt: 232 - 234°C.

**Beispiel 33:**

**3-(1-Methyl-indolyl-3)-5,6-dihydro-2(1 H)-pyrazinon**

240 g (4 Mol) 1,2-Diaminoethan, gelöst in 4000 ml Ethanol, werden vorgelegt und 462 g (2 Mol) 1-Methyl-indolyl-3-glyoxylsäureäthylester bei Raumtemperatur zugetropft. Anschliessend wird 2 Stunden auf 70°C erhitzt. Nach dem Abdampfen des Ethanols wird der Rückstand in 5 Liter Wasser verrührt und mit konzentrierter Salzsäure auf pH 1 gestellt. Vom ungelösten Nebenprodukt wird abfiltriert, das saure Filtrat alkalisch gestellt und der ausfallende Niederschlag abgesaugt.
Ausbeute: 335 g ≈ 73,8 % d. Theorie (Molgewicht 227),
Schmelzpunkt: 176 - 179°C.

**Beispiel 34:**

**3-(1-Methylindolyl-3)-piperazin-2-on:**

22,7 g (0,1 Mol) 3-(1-Methyl-indolyl-3)-5,6-dihydro-2(1 H)-pyrazinon, hergestellt gemäß Beispiel 33, werden, wie in Beispiel 1 beschrieben, hydriert.
Ausbeute: 19,4 g ≈ 85 % d. Theorie (Molgewicht 229),
Schmelzpunkt: 163 - 166°C.

**Beispiel 35:**

**3-(1-Methylindolyl-3)-4-formyl-piperazin-2-on:**

22,9 g (0,1 Mol) 3-(1-Methylindolyl-3)-piperazin-2-on, hergestellt gemäß Beispiel 34, werden, wie in Beispiel 5 beschrieben, mit Ameisensäuremethylester umgesetzt.

Ausbeute 19 g ≈ 78 % d. Theorie (Molgewicht 243)

Schmelzpunkt: 216 - 218°C.

**Beispiel 36:**

**3-[1-(4-Chlorbenzyl)-indolyl-3]-piperazin-2-on:**

164 g (0,68 Mol) 1-(4-Chlorbenzyl)-indol werden in 1400 ml Äther gelöst und 119 g (0,935 Mol) Oxalylchlorid eingetropft. Es wird 8 Stunden bei Raumtemperatur nachgerührt, abgesaugt und der Rückstand 1 Stunde bei 60°C in 800 ml Ethanol gerührt. Nach Einengen und Absaugen wird der Filterrückstand aus Isopropanol umkristallisiert.

Ausbeute: 162,2 g ≈ 70 % d. Theorie *1-(4-Chlorbenzyl-indolyl)-3-glyoxylsäureethylester* (Molgewicht 341), Schmelzpunkt: 123 - 125°C.

136,4 g (0,4 Mol) des nach obiger Vorschrift hergestellten 1-(4-Chlorbenzyl-)indolyl-3-glyoxylsäureethylesters werden, wie in Beispiel 33 beschrieben, mit Ethylendiamin umgesetzt und aufgearbeitet. Man erhält 93 g ≈ 77 % d. Theorie *3-[1-(4-Chlorbenzyl)-indolyl-3]-5,6-dihydro-2(1 H) pyrazinon* vom Schmelzpunkt: 185 - 186°C.

90 g des so erhaltenen Produkts werden, analog den Angaben im Beispiel 1 hydriert und aufgearbeitet. Man erhält 74 g 82 % d. Theorie *3-[1-(4-Chlorbenzyl)-indolyl-3]-piperazin-2-on* vom Schmelzpunkt: 195°C.

**Beispiel 37:**

Führt man das Verfahren des Beispiels 36 analog ausgehend von der gleichen Menge 1-(2-Chlorbenzyl)-indol durch, so erhält man in einer over-all-Ausbeute von 45 % *3[1-(2-Chlorbenzyl-indolyl-3)]-piperazin-2-on*vom Schmelzpunkt: 181°C.

**Beispiel 38:**

**3-(2-Phenylindolyl-3)-piperazin-2-on:**

Die Verbindung wird durch Hydrierung von 3-(2-Phenylindolyl-3)-5,6-dihydro-2(1 H)-pyrazinon in einer Ausbeute von 82 % erhalten. Schmelzpunkt: 165°C (Zersetzung).

Das als Ausgangsmaterial erforderliche 3-(2-Phenylindolyl-3)-5,6-dihydro-2(1 H)-pyrazinon wird hergestellt ausgehend von 2-Phenylindolyl-3-glyoxylsäureethylester und Ethylendiamin nach der in Beispiel 33 beschriebenen Methode.

Ausbeute: 76 % d. Theorie.

Schmelzpunkt: 300°C.

Der für die Umsetzung notwendige 2-Phenylindolyl-3-glyoxylsäureethylester wird aus 2-Phenylindol, Oxalylchlorid und Ethanol, wie in Beispiel 36 beschrieben, in einer Ausbeute von 72 % d. Theorie erhalten.

Schmelzpunkt: 174 - 176°C.

**Beispiel 39:**

**1-Methyl-3-(1-methylindolyl-3)-4-formyl-piperazin-2-on:**

14 g (0,058 Mol) 1-Methyl-3-(1-methyl-indolyl-3)-5,6-dihydro-2(1 H)-pyrazinon werden in 200 ml Methanol gelöst, mit 6,6 g (0,17 Mol) Natriumborhydrid versetzt und 20 Stunden bei 60°C gerührt. Der nach dem Abdampfen des Methanols verbleibende Rückstand wird mit 200 ml Ameisensäuremethylester im Autoklaven 10 Stunden auf 100 - 110°C erhitzt und anschließend eingedampft. Der nach dem Eindampfen zurückbleibende Rückstand wird aus einer Mischung aus Glykol-mono-methyläther und Ethanol umkristallisiert.

Ausbeute: 11 g ≈ 70 % d. Theorie (Molgewicht 271),

Schmelzpunkt: 163 - 165°C.

Das als Ausgangsmaterial erforderliche 1-Methyl-3-(1-methylindolyl-3)-5,6-dihydro-2(1H)pyrazinon wird wie folgt hergestellt:

45,5 g (0,2 Mol) 3-(1-methylindolyl-3)-5,6-dihydro-2(1H)-pyrazinon, hergestellt gemäß Beispiel 33, werden in 400 ml Dimethylformamid gelöst, 5,3 g (0,22 Mol) Natriumhydrid eingetragen, 4 Stunden bei 60°C gerührt, 27,8 g (0,22 Mol) Dimethylsulfat zugetropft und 20 Stunden bei 60°C gerührt. Die nach dem Verdünnen mit Wasser ausfallende Verbindung wird aus Methanol umkristallisiert.

Ausbeute: 22 g ≈ 46 % d. Theorie (Molgewicht 241),
Schmelzpunkt: 165 - 168°C.

**Beispiel 40:**

**3-(1-Methyl-5-methoxy-indolyl-3)-piperazin-2-on:**

Die Verbindung wird hergestellt durch Hydrierung von 3-(1-Methyl-5-methoxy-indolyl-3)-5,6-dihydro-2(1 H)pyrazinon, wie in Beispiel 1 beschrieben.
Ausbeute: 78 % d. Theorie,
Schmelzpunkt: 155 - 157°
Das für diese Herstellung erforderliche Ausgangsmaterial kann wie folgt hergestellt werden:
13,1 g (0,05 Mol) 1-Methyl-5-methoxy-indolyl-3-glyoxylsäure-ethylester werden, wie in Beispiel 33 beschrieben, mit 15 g (0,25 Mol) 1,2-Diaminoethan umgesetzt.
Ausbeute: 8 g ≈ 62 % d. Theorie (Molgewicht 257),
Schmelzpunkt: 225 - 227°C.
Der erforderliche 1-Methyl-5-methoxy-indolyl-3-glyoxylsäure ethylester wird auf folgende Weise hergestellt:
29 g (0,18 Mol) 1-Methyl-5-methoxy-indol werden in 300 ml Äther mit 34,2 g (0,23 Mol) Oxalylchlorid versetzt und 4 Stunden bei Raumtemperatur gerührt. Nach dem Absaugen wird der Rückstand 3 Stunden in 300 ml Ethanol bei 60°C gerührt. Danach wird die Mischung abgesaugt und der Filterrückstand aus Isopropanol umkristallisiert.
Ausbeute: 32 g 68 d. Theorie (Molgewicht 261),
Schmelzpunkt: 121 - 123°C.

**Beispiel 41:**

**3-(4-Methoxyphenyl)-piperazin-2-on:**

20,4 g (0,1 Mol) 3-(4-Methoxyphenyl-)5,6-dihydro-2(1 H)-pyrazinon werden, wie in Beispiel 1 beschrieben, hydriert.
Ausbeute: 17,3 g 84 % d. Theorie (Molgewicht 206),
Schmelzpunkt: 140 - 142°C.

**Beispiel 42:**

**3-[4-(2-Methoxy-ethoxy)phenyl]-piperazin-2-on:**

55,2 g (0,414 Mol) Aluminiumchlorid werden in 150 ml Nitrobenzol gelöst vorgelegt und unter Kühlen 36,8 g (0,27 Mol) Oxalsäureethylesterchlorid und anschließend 63 g (0,414 Mol) 4(2-Methoxy-ethoxy)benzol eingetropft. Die Mischung wird 10 Stunden nachgerührt, auf Eiswasser gegoßen und mit Äther ausgeschüttelt. Nach dem Abtrennen wird die Ätherschicht zuerst mit Natriumhydrogencarbonatlösung, dann mit Wasser gewaschen und getrocknet. Nach dem Eindampfen im Vakuum wird destilliert. Kp.: 150°C 0,1 mbar, Ausbeute: 29 g 4-(2-Methoxy-ethoxy-)phenylglyoxylsäureethylester 28 % d. Theorie (Molgewicht 252).
27,7 g (0,11 Mol) des so hergestellten 4-(2-Methoxy-ethoxy-) phenylglyoxylsäureethylesters werden, wie in Beispiel 33 beschrieben, mit 1,2-Diaminoethan umgesetzt. Man erhält 17 g 62 % d. Theorie 3-[4(-2-Methoxy-ethoxy)phenyl]-5,6-dihydro-2(1 H)pyrazinon (Molgewicht 248),
Schmelzpunkt: 130 - 132°C.
Das so erhaltene Produkt wird anschließend wie in Beispiel 1 beschrieben, hydriert.
Ausbeute: 13,9 g 82 % d. Theorie 3-[4-(2-Methoxyethoxy)-phenyl-]piperazin-2-on,
Schmelzpunkt: 96 - 97°C.

**Beispiel 43:**

**3-(4-Diethylaminoethoxy-phenyl)-piperazin-2-on:**

54 g (0,20 Mol) 4-Hydroxy-phenylglyoxylsäureethylester werden in 500 ml Dimethylformamid gelöst, 46 (0,336 Mol) Kaliumcarbonat und 45 g (0,336 Mol) Diethylaminoethylchlorid zugefügt und 12 Stunden bei Raumtemperatur gerührt. Man gießt das Reaktionsgemisch dann in 3 Liter Wasser, schüttelt mit Essigester aus, trennt die Essigester-Phase ab, trocknet sie und dampft sie im Vakuum ein. Die zurückbleibende ölige Substanz wird in 1000 ml Ethanol gelöst und mit 16 g (0,26 Mol) Ethylendiamin 16 Stunden bei Raumtemperatur gerührt. Dann engt man die Reaktionsmischung im Vakuum ein, verrührt den Rückstand mit Wasser, schüttelt mit Essigester aus und dampft die Essigester-Phase erneut ein. Beim Verreiben des verbleibenden Rückstandes mit Petroläther kristallisiert das Produkt.

Ausbeute: 49,5 g ≈ 61 % d. Theorie (Molgewicht 290),
Schmelzpunkt: 72 - 75°C.

45 g des so erhaltenen *3(4-Diethylaminoethoxy-phenyl)-5,6dihydro-2(1H)-pyrazinons* werden wie in Beispiel 1 beschrieben, hydriert.

Ausbeute: 35,4 g 79 % d. Theorie (Molgewicht 292),
Schmelzpunkt: 82 - 83°C.

**Beispiel 44:**

**3(4-Diethylaminoethoxy-phenyl)-4-formyl-piperazin-2-on:**

29,2 g (0,1 Mol) 3-(4-Diethylaminoethoxy-phenyl-)piperazin-2-on werden, wie in Beispiel 5 beschrieben, durch Kochen mit Ameisensäureethylester acyliert und aufgearbeitet. Nach dem Aufarbeiten wird das Produkt durch Zugabe von Naphthalin1,4-disulfonsäure als Salz dieser Säure gefällt.

Ausbeute: 39,4 g 85 % d. Theorie,
Schmelzpunkt: 150 - 153°C.

**Beispiel 45:**

**3-(3-Pyridyl-)-piperazin-2-on:**

66 g (0,11 Mol) Diaminoäthan werden in 600 ml Ethanol vorgelegt und 17,9 g (0,1 Mol) Pyridyl-3-glyoxylsäureethylester langsam zugetropft. Man rührt 6 - 8 Stunden bei Raumtemperatur nach und hydriert anschließend unter Zusatz von Raney Nickel als Katalysator bei 90°C und 80 bar Wasserstoffdruck bis zur Sättigung. Nach dem Absaugen vom Katalysator dampft man im Vak. zur Trockene und kristallisiert den Rückstand aus Isopropanol um.

Ausbeute: 10,9 gr = 62 % d. Theorie,
Schmelzpunkt: 150 - 152°, Molgewicht 176.

**Beispiel 46**

Tabletten können nach folgender Formulierung hergestellt werden:

| | |
|---|---|
| Wirkstoff | 2 mg |
| Lactose | 60 mg |
| Maisstärke | 30 mg |
| Lösliche Stärke | 4 mg |
| Magnesiumstearat | 4 mg |
| | 100 mg |

**Beispiel 47**

Für die Herstellung von Dragees eignet sich folgende Formulierung:

| | |
|---|---|
| Wirkstoff | 1 mg |
| Maisstärke | 100 mg |
| Lactose | 60 mg |
| sec. Kalziumphosphat | 30 mg |
| Lösliche Stärke | 3 mg |
| Magnesiumstearat | 2 mg |
| Kolloidale Kieselsäure | 4 mg |
| | 200 mg |

**Beispiel 48**

Zur Herstellung von Weichgelatinekapseln mit 5 mg Wirkstoff pro Kapsel eignet sich die folgende Zusammensetzung:

| | |
|---|---|
| Wirkstoff | 5 mg/Kapsel |
| Mischung von Triglyzeriden aus Kokosöl | 150 mg/Kapsel |
| Kapselinhalt | 155 mg |

**Beispiel 49**

Injectionslösungen mit 1 mg Wirkstoff pro ml können nach folgender Rezeptur hergestellt werden:

| | |
|---|---|
| Wirkstoff | 1,0 mg |
| Polyethylenglycol 400 | 0,3 mg |
| Natriumchlorid | 2,7 mg |
| Wasser für Injections-zwecke | auf 1 ml |

**Beispiel 50**

Emulsionen mit 3 mg Wirkstoff per 5 ml können nach folgender Rezeptur hergestellt werden:

| | |
|---|---|
| Wirkstoff | 0,06 g |
| Neutralöl | q.s. |
| Na.Carboxymethylzellulose | 0,6 g |
| Polyoxyethylenstearat | q.s. |
| Reinglyzerin | 0,2 bis 2 g |
| Aromastoffe | q.s |
| Wasser (entmineralisiert oder distilliert) | auf 100 ml |

Die Prüfung der erfindungsgemäßen Verbindungen erfolgt durch verschiedene Testverfahren A, B und C, die jedes für sich, insbesondere aber in ihrer Kombination, die Wirksamkeit und den Wert der Substanzen zeigen.

**A) "Nitrithypoxie"**

In diesem Test wird nach der Methode von Gibson und Blass (J. Neurochem. *27*, 1976) bei Mäusen mit $NaNO_2$ (250 mg/kg s.) eine cerebrale Hypoxie erzeugt, die mit dem Tod der Versuchstiere endet. Bestimmt wird, ob die Überlebenszeit durch Prämedikation mit der Testsubstanz beeinflußt werden kann.

Als minimale aktive Dosis bezeichnet man diejenige, die zu einer signifikanten Verlängerung der Überlebenszeit (t-Test) der Versuchtstiere führt.

**B) "Stickstoffhypoxie"**

Zur Bestimmung der Einflüsse einer Stickstoff-Beatmung auf die elektrische Aktivität des Gehirns werden Ratten mit chronisch implantierten Elektroden zur Messung des Elektroencephalogramms (EEG) eingesetzt.

Eine Stunde nach Verabreichung von Kontroll- und Präparatinjektion werden die Tiere in Hexobarbital-Narkose unter EEG-Kontrolle mit Stickstoff beatmet. Als Hypoxie-Toleranz wird die Zeitspanne zwischen Beginn der Beatmung und dem völligen Verschwinden der elektrischen Aktivität (isoelektrisches EEG) bezeichnet. Eine kurzdauernde weitere Beatmung mit Stickstoff und anschließender Luftbeatmung führt üblicherweise zum Tod aller Kontrolltiere.

Als minimale aktive Dosis eines Präparats bezeichnet man diejenige, die zu einer signifikanten Verlängerung der Hypoxie-Toleranz (t-Test) führt und bzw. oder die Letalität signifikant (Chi-Quadrat-Test) vermindert.

**C) "Passive avoidance"**

Die Testapparatur ist eine Hell-Dunkel-Box mit elektrifizierbarem Gitterboden im dunklen Teil.

90 Minuten nach Verabreichung von Kontroll- und Präparatinjektion werden unerfahrene männliche Mäuse mit Scopolaminhydrobromid (3 mg/kg i.p.) behandelt. 5 Minuten später werden die Mäuse in den hellen Teil der Box gesetzt. Nach dem Überwechseln in den dunklen Teil der Box erhalten sie einen ihnen unangenehmen elektrischen Schlag. Nach 24 Stunden wird jede Maus einmal in den hellen Teil der Testapparatur gesetzt und die Verweildauer (max. 300 sec) gemessen. Die signifikante Wirkung der Testsubstanz im Vergleich zur Kontrollgruppe wird mittels Mediantest berechnet.

Als minimale aktive Dosis eines Präparats bezeichnet man diejenige, die eine signifikante Wirkung gegen Scopolamin zeigt. Die mit aktiver Dosis eines Präparates und Scopolamin behandelten Tiere zeigen eine lange Verweildauer, ebenso die nicht mit Scopolamin behandelten Tiere, wogegen diejenigen mit Kontrollinjektion und Scopolamin behandelten eine kurze Verweildauer zeigen.

Die Tabelle I auf Seite 36 zeigt, daß die erfindungsgemäßen Verbindungen gegenüber den bekannten Vergleichssubstanzen ein vollständig neues, cerebral-protektives Profil aufweisen, da sie in allen drei Testmodellen signifikant wirksam sind.

**Tabelle I**

| Substanz | | | Minimale aktive Dosis/mg/kg/ | | | |
|---|---|---|---|---|---|---|
| $R^1$ | $R^2$ | $R^3$ | Nitrithypoxie | Stickstoff Toleranz | hypoxie Letalität | Passive avoidance |
| 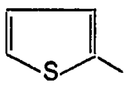 | H | H | 50 p.o. | 200 i.p. | 100 i.p. | 12,5 p.o. |
| 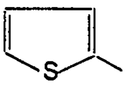 | 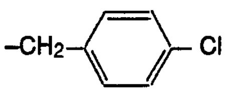 | H | 400 p.o. | 200 i.p. | 100 i.p. | 100 p.o. |
| Piracetam | | | 125 p.o. | 500 i.p. | - | - |
| Pyritinol | | | - | - | 200 i . p. | - |
| Meclofenoxat | | | 300 p.o. | | - | - |
| Vincamin | | | - | - | - | 50 p.o. |

Den folgenden Tabellen II und III sind weitere pharmakologischen Daten zu entnehmen.

Die erfindungsgemäßen Vorbindungen zeigen eine starke Wirkung in niedriger Dosierung, eine gute Verträglichkeit sowie eine geringe Toxizität.

**Tabelle II, Nitrithypoxie**

| R1 | Substanz R2 | R3 | Min. akt. Dos. [mm/kg] |
|---|---|---|---|
| 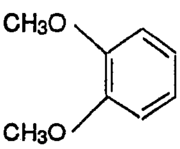 | -CO-$CH_3$ | -$CH_2$-CO-$NH_2$ | 100 p.o. |
| 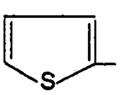 | 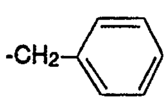 | 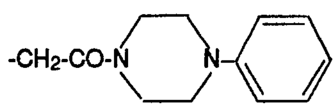 | 100 p.o. |
| 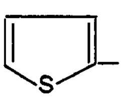 | -$CH_2$-$CH_2$OH | H | 100 p.o. |
| " | 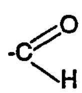 | H | 100 p.o. |
| " | 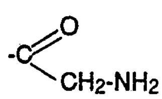 | H | 100 p.o. |

| Substanz R1 | R2 | R3 | Min. akt. Dos. [mm/kg] |
|---|---|---|---|
| 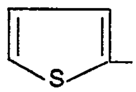 | 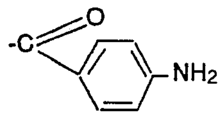 | H | 100 p.o |
| " | 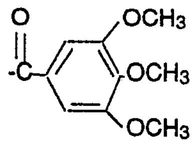 | H | 100 p.o. |
| " | 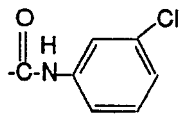 | H | 100 p.o. |
| " | 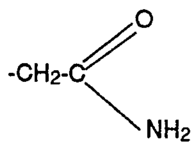 | H | 50 p.o. |
| " | 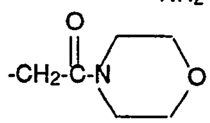 | H | 100 p.o. |
| " | 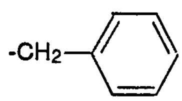 | H | 100 p.o. |
| " | 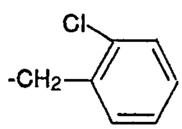 | H | 100 p.o. |
| " | 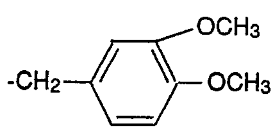 | H | 25 p.o. |
| " | 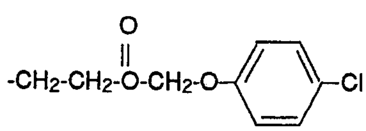 | H | 100 p.o. |
| " | $-CH_2-CH=CH_2$ | H | 100 p.o. |
| " | $-CH_2-C\equiv CH$ | H | 100 p.o. |
| 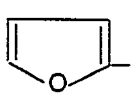 | -H | H | 100 p.o. |
| " | $-CH_2-CH_2-OH$ | H | 100 p.o. |
| " | 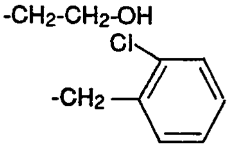 | H | 100 p.o. |

| R1 | Substanz R2 | R3 | Min. akt. Dos. [mm/kg] |
|---|---|---|---|
| 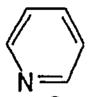 | -H | H | 100 p.o. |
| 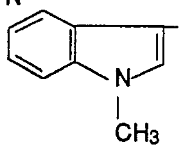 | -H | H | 100 p.o. |
| " | $-CH_2-CH_2-OH$ | H | 100 p.o. |
| " | 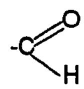 | H | 100 p.o. |
| " | 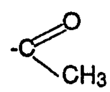 | H | 100 p.o. |
| " | 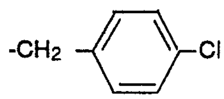 | H | 100 p.o. |
| " | 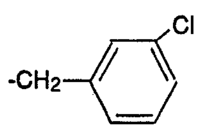 | H | 100 p.o. |
| " | 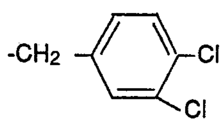 | H | 100 p.o. |
| " | 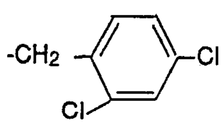 | H | 100 p.o. |
| Piracetam | | | 125 p.o. |
| Meclofenoxat | | | 300 p.o. |

**Tabelle III, Stickstoffhypoxie**

| R1 | Substanz R2 | R3 | Min. akt. Hypoxie-Toleranz | Dos. [mg/kg] Letalität |
|---|---|---|---|---|
| 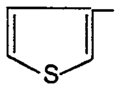 | -CH2-CH2-OH | H | 200 i.p. | 200 i.p. |
| " | 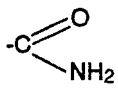 | H | 200 i.p. | 100 i.p. |
| " | 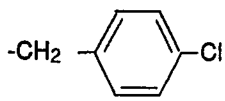 | H | 200 i.p. | 100 i.p. |
| " | 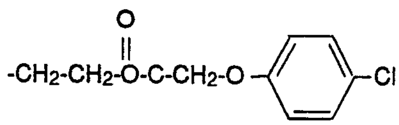 | H | 300 i.p. | 200 i.p. |
| 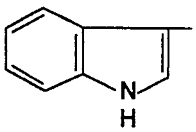 | 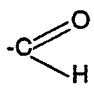 | H | 200 i.p. | 200 i.p. |
| 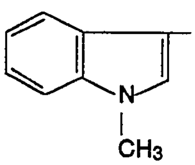 | 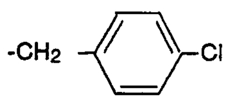 | H | 200 i.p. | 200 i.p. |
| " | 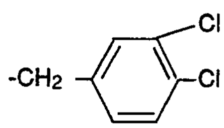 | H | 200 i.p. | 200 i.p. |
| Piracetam | | | 500 i.p. | - |
| Pyritinol | | | - | 200 i.p. |

**Patentansprüche** für die Vertragsstaaten: BE, CH, DE, FR, GB, IT, LI, NL, SE

1. Substituierte Piperazin-2-one der allgemeinen Formel I

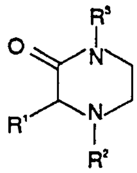

(I)

worin

$R^1$ durch Alkoxy mit 1 bis 4 C-Atomen, Fluor, Alkoxyalkyl mit insgesamt 2 bis 6 C-Atomen oder Dialkylaminoalkoxy mit insgesamt 4 bis 8 C-Atomen substituiertes Phenyl; 2-Thienyl; 3-Pyridyl oder einen Rest der Formel II

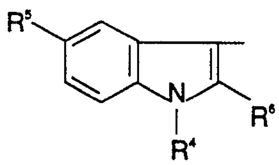

(II)

$R^2$ Wasserstoff; Alkyl mit 1 bis 4 C-Atomen, gegebenenfalls substituiert durch Alkoxycarbonyl mit insgesamt 2 bis 5 Kohlenstoffatomen, oder durch Aminocarbonyl, wobei die Aminogruppe des Aminocarbonylrestes ihrerseits 1- oder 2-fach durch Methyl oder Ethyl substituiert sein kann, oder Glied eines Piperidin-, Piperazin- oder Morpholinringes ist; Phenylalkyl mit 1 bis 3 C-Atomen im Alkylrest, gegebenenfalls substituiert im Phenylkern durch Chlor oder Alkoxy mit 1 oder 2 C-Atomen oder Hydroxy; Alkanoyl mit 1 bis 4 C-Atomen, das gegebenenfalls durch Chlor substituiert ist; Nicotinoyl; Benzoyl, gegebenenfalls substituiert durch Alkoxy mit 1 oder 2 C-Atomen, Nitro oder Amino; Aminosulfonyl; Aminosulfonyl, das am N durch 1 oder 2 Alkylgruppen mit je 1 bis 4 C-Atomen substituiert ist, oder Aminosulfonyl, dessen Aminogruppe Glied eines Piperidin-, Piperazin-, Morpholin- oder 4-Alkyl-piperazin-Ringes ist, wobei der 4-Alkyl-piperazinring 1 bis 4 C-Atome im Alkylrest aufweist; einen Rest der Formel III

$$-\underset{\underset{O}{\|}}{C}-CH_2-R^7 \qquad (III);$$

einen Rest der Formel IV

$$-\underset{\underset{O}{\|}}{C}-NH-R^8 \qquad (IV);$$

einen Rest der Formel V

$$-CH_2-CH_2-OR^9 \qquad (V);$$

$R^3$ Wasserstoff; Alkyl mit 1 bis 4 C-Atomen; Alkoxycarbonylmethyl mit insgesamt 3 bis 6 C-Atomen; Aminocarbonylmethyl; N-mono- oder disubstituiertes Aminocarbonylmethyl mit insgesamt 3 bis 5 C-Atomen oder Diethylaminocarbonylmethyl; oder eine Gruppe der Formel VII

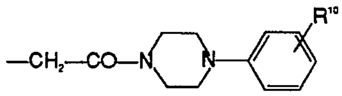

(VII);

| | |
|---|---|
| $R^4$ | Wasserstoff; Alkyl mit 1 bis 4 C-Atomen; Phenalkyl mit 1 oder 2 C-Atomen im Alkylrest, das gegebenenfalls durch Chlor substituiert ist; |
| $R^5$ | Wasserstoff oder Alkoxy mit 1 bis 4 C-Atomen; |
| $R^6$ | Wasserstoff oder Phenyl; |
| $R^7$ | Amino; Piperidin-1-yl; Piperazin-1-yl; Morpholin-1-yl oder 4-Alkylpiperazin-1-yl mit bis 4 C-Atomen im Alkylrest; |
| $R^8$ | Wasserstoff; gegebenenfalls durch Chlor substituiertes Phenyl; |
| $R^9$ | Wasserstoff; Phenoxyacetyl oder einen Rest der Formel VI |

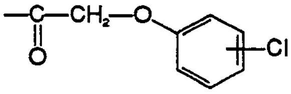

(vi)

| | |
|---|---|
| $R^{10}$ | Wasserstoff oder Alkoxy mit 1 bis 4 C-Atomen bedeuten und ihre physiologisch verträglichen Säureadditionssalze. |

2. Substituiertes Piperazin-2-on nach Anspruch 1 dadurch gekennzeichnet, daß

| | |
|---|---|
| $R^1$ | 2-Thienyl; |
| $R^2$ | Wasserstoff; |
| $R^3$ | Wasserstoff |
| | bedeuten. |

3. 3-(4-(2Methoxy-ethoxy)-phenyl)-piperazin-2-on.

4. Substituierte Piperazinone der Formel Ia

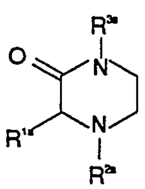

(Ia)

worin

| | |
|---|---|
| $R^{1a}$ | 2-Furyl; |
| $R^{2a}$ | Wasserstoff, 2-Hydroxyethyl oder 2-Chlor-benzyl und |
| $R^{3a}$ | Wasserstoff |
| | bedeuten. |

5. Substituierte Piperazinone der Formel Ib

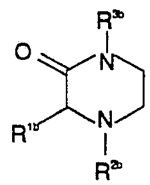

(Ib)

worin

| | |
|---|---|
| $R^{1b}$ | 2-Thienyl, |
| $R^{2b}$ | Allyl, oder Propargyl und |
| $R^{3b}$ | Wasserstoff |
| | bedeuten. |

6. Verfahren zur Herstellung der in den Ansprüchen 1 bis 5 angegebenen substituierten Piperazin-2-one der Formel I, dadurch gekennzeichnet, daß man ein 5,6-Dihydro-2(1H)-pyrazinon der Formel VIII

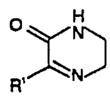

(VIII)

worin $R^1$ die in den Ansprüchen 1 bis 5 angegebene Bedeutung besitzt, zu dem entsprechenden substituierten Piperazin-2-on der allgemeinen Formel I, wobei $R^2$ und $R^3$ Wasserstoff bedeuten, reduziert und gegebenenfalls danach mit einem Alkylierungs- bzw. Acylierungsmittel der Formel IX

$X\text{–}R^2$ (IX)

worin X ein anionisch abspaltbarer Rest ist und $R^2$ die in den Ansprüchen 1 bis 5 angegebene Bedeutung besitzt, in an sich bekannter Weise umsetzt und/oder gegebenenfalls das erhaltene Piperazin-2-on der Formel I, wobei $R^3$ Wasserstoff ist, mit einem Alkylierungsmittel der Formel X

$X\text{–}R^3$ (X)

worin X ein anionisch abspaltbarer Rest ist und $R^3$ die in den Ansprüchen 1 bis 5 angegebene Bedeutung besitzt, in an sich bekannter Weise umsetzt und das erhaltene substituierte Piperazin-2-on gewünschtenfalls in an sich bekannter Weise in ein physiologisch verträgliches Säureadditionssalz überführt.

7. Verwendung eines substituierten Piperazin-2-ons oder eines seiner physiologisch verträglichen Säureadditionssalze eines oder mehrerer der Ansprüche 1 bis 5 zur Herstellung eines Arzneimittels für die Verbesserung der Hirnfunktion.

8. Verwendung eines substituierten Piperazin-2-ons oder eines seiner physiologisch verträglichen Säureadditionssalze eines oder mehrerer der Ansprüche 1 bis 5 zur Herstellung nootropisch wirkender Arzneimittelzubereitungen.

**Patentansprüche** für den Vertragsstaat: AT

1. Verfahren zur Herstellung substituierter Piperazin-2-one der allgemeinen Formel I

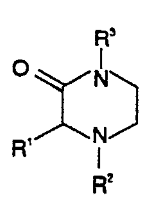

(I)

worin

$R^1$ durch Alkoxy mit 1 bis 4 C-Atomen, Fluor, Alkoxyalkyl mit insgesamt 2 bis 6 C-Atomen oder Dialkylaminoalkoxy mit insgesamt 4 bis 8 C-Atomen substituiertes Phenyl; 2-Thienyl; 3-Pyridyl oder einen Rest der Formel II

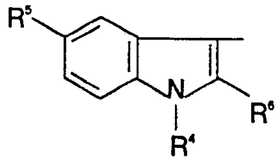

(II);

$R^2$ Wasserstoff; Alkyl mit 1 bis 4 C-Atomen, gegebenenfalls substituiert durch Alkoxycarbonyl mit insgesamt 2 bis 5 Kohlenstoffatomen, oder durch Aminocarbonyl, wobei die Aminogruppe des Aminocarbonylrestes ihrerseits 1- oder 2-fach durch Methyl oder Ethyl substituiert sein kann, oder Glied eines Piperidin-, Piperazin- oder Morpholinringes ist; Phenylalkyl mit 1 bis 3 C-Atomen im Alkylrest, gegebenenfalls substituiert im Phenylkern durch Chlor oder Alkoxy mit 1 oder 2 C-Atomen oder Hydroxy; Alkanoyl mit 1 bis 4 C-Atomen, das gegebenenfalls durch Chlor substituiert ist; Nicotinoyl; Benzoyl, gegebenenfalls substituiert durch Alkoxy mit 1 oder 2 C-Atomen, Nitro oder Amino; Aminosulfonyl; Aminosulfonyl, das am N durch 1 oder 2 Alkylgruppen mit je 1 bis 4 C-Atomen substituiert ist, oder Aminosulfonyl, dessen Aminogruppe Glied eines Piperidin-, Piperazin-, Morpholin- oder 4-Alkyl-piperazin-Ringes ist, wobei der 4-Alkyl-piperazinring 1 bis 4 C-Atome im Alkylrest aufweist; einen Rest der Formel III

$$-\underset{\underset{O}{\|}}{C}-CH_2-R^7 \qquad (III);$$

einen Rest der Formel IV

$$-\underset{\underset{O}{\|}}{C}-NH-R^8 \qquad (IV);$$

einen Rest der Formel V

$$-CH_2-CH_2-OR^9 \qquad (V);$$

$R^3$ Wasserstoff; Alkyl mit 1 bis 4 C-Atomen; Alkoxycarbonylmethyl mit insgesamt 3 bis 6 C-Atomen; Aminocarbonylmethyl; N-mono- oder disubstituiertes Aminocarbonylmethyl mit insgesamt 3 bis 5 C-Atomen oder Diethylaminocarbonylmethyl; oder eine Gruppe der Formel VII

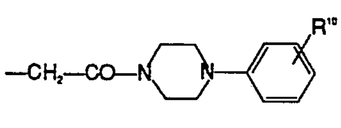

(VII);

$R^4$ Wasserstoff; Alkyl mit 1 bis 4 C-Atomen; Phenalkyl mit 1 oder 2 C-Atomen im Alkylrest, das gegebenenfalls durch Chlor substituiert ist;

$R^5$ Wasserstoff oder Alkoxy mit 1 bis 4 C-Atomen;

$R^6$ Wasserstoff oder Phenyl;

$R^7$ Amino; Piperidin-1-yl; Piperazin-1-yl; Morpholin-1-yl oder 4-Alkylpiperazin-1-yl mit 1 bis 4 C-Atomen im Alkylrest;

$R^8$ Wasserstoff; gegebenenfalls durch Chlor substituiertes Phenyl;

$R^9$ Wasserstoff; Phenoxyacetyl oder einen Rest der Formel VI

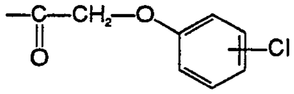

(VI)

$R^{10}$ Wasserstoff oder Alkoxy mit 1 bis 4 C-Atomen bedeuten und ihre physiologisch verträglichen Säureadditionssalze,

dadurch gekennzeichnet, daß ein 5,6-Dihydro-2(1H)-pyrazin-on der Formel VIII

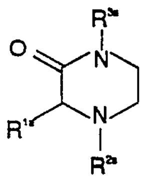

(VIII)

zu dem entsprechenden substituierten Piperazin-2-on der allgemeinen Formel I, wobei $R^2$ und $R^3$ Wasserstoff bedeuten, reduziert und gegebenenfalls danach mit einem Alkylierungs- bzw. Acylierungsmittel der Formel IX

$X–R^2$ (IX);

worin X ein anionisch abspaltbarer Rest ist, in an sich bekannter Weise umgesetzt und/oder gegebenenfalls das erhaltene Piperazin-2-on der Formel I, wobei $R^3$ Wasserstoff ist, mit einem Alkylierungsmittel der Formel X

$X–R^3$ (X);

worin X ein anionisch abspaltbarer Rest ist, in an sich bekannter Weise umgesetzt und das erhaltene substituierte Piperazin-2-on gewünschtenfalls in an sich bekannter Weise in ein physiologisch verträgliches Säureadditionssalz überführt wird.

2. Verfahren nach Anspruch 1, dadurch gekennzeichnet, daß die Ausgangsverbindungen so ausgewählt werden, daß eine Verbindung der Formel I entsteht, wobei

$R^1$ 2-Thienyl;
$R^2$ Wasserstoff;
$R^3$ Wasserstoff
bedeuten.

3. Verfahren zur Herstellung von 3-(4-(2-Methoxy-ethoxy)-phenyl)-piperazin-2-on, dadurch gekennzeichnet, daß man 3-(4-(2-Methoxy-ethoxy)phenyl)-5,6-dihydro-2(1H)-pyrazinon hydriert.

4. Verfahren zur Herstellung substituierter Piperazinone der Formel Ia

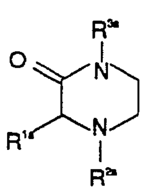

(Ia)

worin

$R^{1a}$ 2-Furyl;
$R^{2a}$ Wasserstoff, 2-Hydroxyethyl oder 2-Chlor-benzyl und

$R^{3a}$ Wasserstoff

bedeuten, dadurch gekennzeichnet, daß man 3-(2-Furyl)-5,6-dihydro-2(1H)pyrazinon hydriert und gegebenenfalls danach mit einem Alkylierungsmittel der Formel IXa

$X–R^{2a}$ (IXa)

worin X ein anionisch abspaltbarer Rest ist, in an sich bekannter Weise umsetzt.

5. Verfahren zur Herstellung substituierter Piperazinone der Formel Ib

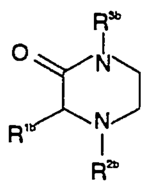

(Ib)

worin

$R^{1b}$ 2-Thienyl,
$R^{2b}$ Allyl oder Propargyl und
$R^{3b}$ Wasserstoff

bedeuten, dadurch gekennzeichnet, daß man 3-(2-Thienyl)-5,6-dihydro-2(1H)pyrazinon hydriert und danach mit einem Alkylierungsmittel der Formel IXb

$X–R^{2b}$ (Ixb)

worin X ein anionisch abspaltbarer Rest ist, in an sich bekannter Weise umsetzt.

6. Verwendung eines nach dem Verfahren der Ansprüche 1 bis 5 hergestellten substituierten Piperazin-2-ons oder eines seiner physiologisch verträglichen Säureadditionssalze zur Herstellung eines Arzneimittels für die Verbesserung der Hirnfunktion.

7. Verwendung eines nach dem Verfahren der Ansprüche 1 bis 5 hergestellten substituierten Piperazin-2-ons oder eines seiner physiologisch verträglichen Säureadditionssalze zur Herstellung nootropisch wirkender Arzneimittelzubereitungen.

**Claims** for the Contracting States: BE, CH, DE, FR, GB, IT, LI, NL, SE

1. Substituted piperazin-2-ones of the general formula I

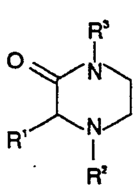

(I);

wherein

$R^1$ denotes phenyl which is substituted by alkoxy having 1 to 4 C atoms, fluorine, alkoxyalkyl having a total of 2 to 6 C atoms or dialkylaminoalkoxy having a total of 4 to 8 C atoms; 2-thienyl; 3-pyridyl or a radical of the formula II

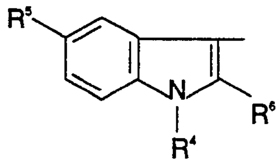

(II);

$R^2$ denotes hydrogen; alkyl having 1 to 4 C atoms and being optionally substituted by alkoxycarbonyl having a total of 2 to 5 C atoms or aminocarbonyl, it being possible for the amino group of the aminocarbonyl radical to be monosubstituted or disubstituted by methyl or ethyl or is part of a piperidine, piperazine or morpholine nucleus; phenalkyl having 1 to 3 C atoms in the alkyl radical and being optionally substituted in the phenyl nucleus by chlorine or alkoxy having 1 or 2 C atoms or hydroxyl; alkanoyl having 1 to 4 C atoms and being optionally substituted by chlorine; nicotinoyl; benzoyl which is optionally substituted by alkoxy having 1 or 2 C atoms, nitro or amino; aminosulphonyl or aminosulphonyl, the nitrogen of which is substituted by 1 or 2 alkyl groups each having 1 to 4 C atoms, or aminosulphonyl, the amino group of which is part of a piperidine, piperazine, morpholine or 4-alkyl-piperazine nucleus, the 4-alkyl-piperazine nucleus having 1 to 4 C atoms in the alkyl radical; a radical of the formula III

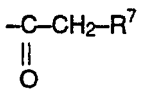

(III);

a radical of the formula IV

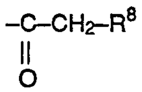

(IV);

a radical of the formula V

$-CH_2-CH_2-OR^9$ (V);

$R^3$ denotes hydrogen; alkyl having 1 to 4 C atoms; alkoxycarbonylmethyl having a total of 3 to 6 C atoms; aminocarbonylmethyl which is optionally N-monosubstituted or N-disubstituted and has a total of 3 to 5 C atoms or diethylaminocarbonylmethyl; or a group of the formula VII

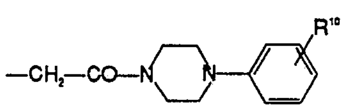

(VII);

$R^4$ is hydrogen; Alkyl having 1 to 4 C atoms; phenalkyl which has 1 or 2 C atoms in the alkyl radical and is optionally substituted by chlorine;

$R^5$ is hydrogen or alkoxy having 1 to 4 C atoms;

$R^6$ is hydrogen or phenyl;

$R^7$ is amino; piperidin-1-yl; piperazin-1-yl; morpholin-1-yl or 4-alkylpiperazin-1-yl having 1 to 4 C atoms in the alkyl radical;

$R^8$ is hydrogen; phenyl or phenyl which is substituted by chlorine;

$R^9$ is hydrogen; phenoxyacetyl or a radical of the formula VI

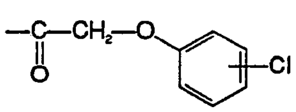

(VI);

$R^{10}$ is hydrogen or alkoxy having 1 to 4 C atoms and their physiologically acceptable acid addition salts.

2. Substituted piperazin-2-one according to Claim 1, characterized in that

$R^1$ denotes 2-thienyl;
$R^2$ denotes hydrogen;
$R^3$ denotes hydrogen.

3. 3-(4-(2-Methoxy-ethoxy)-phenyl)-piperazin-2-one.

4. Substituted piperazinones of the formula Ia

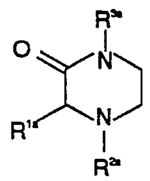

(Ia)

wherein

$R^{1a}$ denotes 2-furyl;
$R^{2a}$ denotes hydrogen, 2-Hydroxyethyl or 2-chlorobenzyl and
$R^{3a}$ denotes hydrogen.

5. Substituted piperazinones of the formula Ib

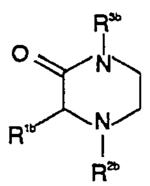

(Ib)

wherein

$R^{1b}$ denotes 2-thienyl;
$R^{2b}$ denotes allyl or propargyl and
$R^{3b}$ denotes hydrogen.

6. Process for the preparation of the substituted piperazin-2-ones of the formula I and indicated in Claims 1 to 5, characterized in that a 5,6-dihydro-2(1H)-pyrazinone of the formula VIII

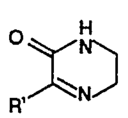

wherein

$R^1$ has the meaning indicated in Claims 1 to 5, is reduced to give the corresponding substituted piperazin-2-one of the general formula I in which $R^2$ and $R^3$ are hydrogen, this piperazin-2-one is, if appropriate, then reacted, in a manner which is in itself known, with an alkylating or acylating agent of the formula IX

$X-R^2$ (IX)

wherein X is a radical which can be removed as an anion, and $R^2$ has the meaning indicated in Claims 1 to 5 and, if appropriate, the resulting piperazin-2-one of the formula I in which $R^3$ is hydrogen is then reacted, in a manner which is in itself known, with an alkylating agent of the formula X

$X-R^3$ (X)

wherein X is a radical which can be removed as an anion, and $R^3$ has the meaning indicated in Claims 1 to 5, and the resulting substituted piperazin-2-one is, if desired, converted in a manner which is in itself known to give a physiologically acceptable acid addition salt.

7. Use of a substituted piperazin-2-one or one of its physiologically acceptable acid addition salts of one or more of claims 1 to 5 for preparing a medicinal formulation for ameliorating the cerebral function.

8. Use of a substituted piperazin-2-one or one of its physiologically acceptable acid addition salts of one or more of Claims 1 to 5 for preparing medicinal formulations which have a nootropic action.

**Claims** for the contracting State: AT

1. Process for preparing substituted piperazin-2-ones of the general formula I

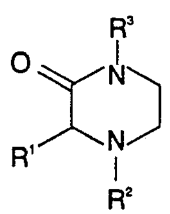

wherein

R1 denotes phenyl which is substituted by alkoxy having 1 to 4 C atoms, fluorine, alkoxyalkyl having a total of 2 to 6 C atoms or dialkylaminoalkoxy having a total of 4 to 8 C atoms; 2-thienyl; 3-pyridyl or a radical of the formula II

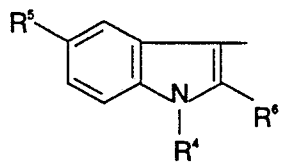

$R^2$ denotes hydrogen; alkyl having 1 to 4 C atoms and being optionally substituted by alkoxycarbonyl having a total of 2 to 5 C atoms or aminocarbonyl, it being possible for the amino group of the aminocarbonyl radical to be monosubstituted or disubstituted by methyl or ethyl or is part of a piperidine, piperazine or morpholine nucleus; phenalkyl having 1 to 3 C atoms in the alkyl radical and being optionally substituted in the phenyl nucleus by chlorine or alkoxy having 1 or 2 C atoms or hydroxyl; alkanoyl having 1 to 4 C atoms and being optionally substituted by chlorine; nicotinoyl; benzoyl which is optionally substituted by alkoxy having 1 or 2 atoms, nitro or amino; aminosulphonyl or aminosulphonyl, the nitrogen of which is substituted by 1 or 2 alkyl groups each having 1 to 4 C atoms, or aminosulphonyl, the amino group of which is part of a piperidine, piperazine, morpholine or 4-alkyl-piperazine nucleus, the 4-alkylpiperazine nucleus having 1 to 4 C atoms in the alkyl radical; a radical of the formula III

$-C-CH_2-R^7$
||
O (III);

a radical of the formula IV

$-C-CH_2-R^8$
||
O (IV);

a radical of the formula V

$-CH_2-CH_2-OR^9$ (V);

$R^3$ denotes hydrogen; alkyl having 1 to 4 C atoms; alkoxycarbonylmethyl having a total of 3 to 6 C atoms; aminocalbonylmethyl; aminocarbonylmethyl which is N-monosubstituted or N-disubstituted and has a total of 3 to 5 C atoms or diethylaminocarbonylmethyl; or a group of the formula VII

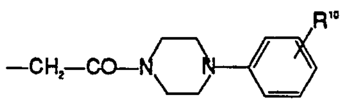
(VII);

$R^4$ is hydrogen; Alkyl having 1 to 4 C atoms; phenalkyl which has 1 or 2 C atoms in the alkyl radical and is optionally substituted by chlorine;
$R^5$ is hydrogen or alkoxy having 1 to 4 C atoms;
$R^6$ is hydrogen or phenyl;
$R^7$ is amino; piperidin-1-yl; piperazin-1-yl; morpholin-1-yl or 4-alkylpiperazin-1-yl having 1 to 4 C atoms in the alkyl radical;
$R^8$ is hydrogen; phenyl or phenyl which is substituted by chlorine;
$R^9$ is hydrogen; phenoxyacetyl or a radical of the formula VI

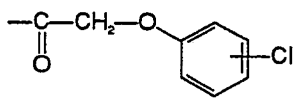
(VI);

$R^{10}$ is hydrogen or alkoxy having 1 to 4 C atoms and their physiologically acceptable acid addition salts, characterized in that a 5,6-dihydro-2(1H)-pyrazinone of the formula VIII

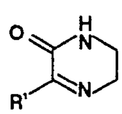
(VIII)

is reduced to give the corresponding substituted piperazin-2-one of the general formula I in which $R^2$ and
$R^3$ are hydrogen, this piperazin-2-one is, if appropriate, then reacted, in a manner which is in itself known, with an alkylating or acylating agent of the formula IX

$X-R^2$ (IX),

wherein X is a radical which can be removed as an anion, and, if appropriate, the resulting piperazin-2-one of the formula I in which $R^3$ is hydrogen is then reacted, in a manner which is in itself known, with an alkylating agent of the formula X

X – $R^3$ (X),

wherein X is a radical which can be removed as an anion, and the resulting substituted piperazin-2-one is, if desired, converted in a manner which is in itself known to give a physiologically acceptable acid addition salt.

2. Process according to Claim 1, characterized in that the starting compounds are selected such that a compound of formula I is formed wherein

$R^1$ denotes 2-thienyl;
$R^2$ denotes hydrogen;
$R^3$ denotes hydrogen.

3. Process for preparing 3-(4-(2-methoxy-ethoxy)-phenyl)-piperazin-2-one, characterized in that 3-(4-(2-methoxy-ethoxy)phenyl)-5,6-dihydro-2(1H)-pyrazinone is hydrated.

4. Process for preparing substituted piperazinones of the formula Ia

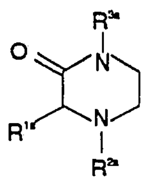

(Ia)

wherein

$R^{1a}$ denotes 2-furyl;
$R^{2a}$ denotes hydrogen, 2-Hydroxyethyl or 2-chlorobenzyl and
$R^{3a}$ denotes hydrogen, characterized in that 3-(2-furyl)-5,6-dihydro-2(1H)pyrazinone is hydrated and, if appropriate, is then reacted, in a manner which is in itself known, with an alkylating agent of the formula IXa

X - $R^{2a}$ (IXa)

wherein X is a radical which can be removed as an anion.

5. Process for preparing substituted piperazinones of the formula Ib

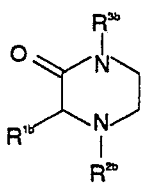

(Ib)

wherein

$R^{1b}$ denotes 2-thienyl;
$R^{2b}$ denotes allyl or propargyl and
$R^{3b}$ denotes hydrogen,
characterized in that 3-(2-thienyl)-5,6-dihydro-2(1H)-pyrazinone is hydrated and is then reacted, in a manner which is in itself known, with an alkylating agent of the formula IXb

X–$R^{2b}$ (IXb)

wherein X is a radical which can be removed as an anion.

6. Use of a substituted piperazin-2-one or one of its physiologically acceptable acid addition salts prepared according to the process of claims 1 to 5 for preparing a medicinal formulation for ameliorating the cerebral function.

7. Use of a substituted piperazin-2-one or one of its physiologically acceptable acid addition salts prepared according to the process of claims 1 to 5 for preparing medicinal formulations which have a nootropic action.

**Revendications** pour les Etats Contractants: BE, CH, DE, FR, GB, IT, LI, NL, SE

1. Pipérazinones-2 substituées qui répondent à la formule générale I:

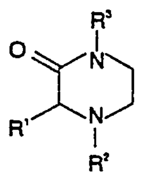

(I)

dans laquelle:

$R^1$ représente un phényle porteur d'un alcoxy en $C_1$-$C_4$, le fluor un alcoxy-alkyle contenant au total de 2 à 6 atomes de carbone ou un dialkylamino-alcoxy contenant au total de 6 à 8 atomes de carbone; un thiényle-2; un pyridyle-3 ou un radical de formule II:

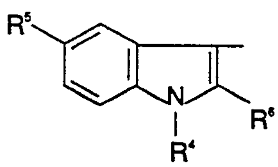

(II);

$R^2$ représente l'hydrogène; un alkyle en $C_1$-$C_4$, éventuellement porteur d'un alcoxycarbonyle contenant au total de 2 à 5 atomes de carbone, ou d'un aminocarbonyle dont l'amino peut lui-même porter un ou deux substituants pris dans l'ensemble constitué par le méthyle et l'éthyle ou fait partie d'un noyau de pipéridine, de pipérazine ou de morpholine; un phénylalkyle dont l'alkyle contient de 1 à 3 atomes de carbone et dont le noyau phényle porte éventuellement du chlore, un alcoxy à un ou deux atomes de carbone ou un hydroxy; un alcanoyle en $C_1$-$C_4$, éventuellement porteur de chlore; un nicotinoyle; un benzoyle éventuellement porteur d'un alcoxy à un ou deux atomes de carbone, d'un nitro ou d'un amino; un aminosulfonyle; un aminosulfonyle dont l'azote porte un ou deux alkyles contenant chacun de 1 à 4 atomes de carbone, ou un aminosulfonyle dont le radical amino fait partie d'un cycle de pipéridine, de pipérazine, de morpholine ou d'alkyl-4 pipérazine, l'alkyle en 4 de cette dernière contenant de 1 à 4 atomes de carbone; un radical de formule III:

$$-\underset{\underset{O}{\|}}{C}-CH_2-R^7 \qquad (III);$$

un radical de formule IV:

$$-\underset{\underset{O}{\|}}{C}-NH-R^8 \qquad (IV);$$

ou un radical de formule V:

$$-CH_2-CH_2-OR^9 \qquad (V);$$

$R^3$ représente l'hydrogène; un alkyle en $C_1$-$C_4$; un alcoxycarbonylméthyle contenant au total de 3 à 6 atomes de carbone; un aminocarbonylméthyle; un aminocarbonylméthyle mono ou di-substitué à l'azote et contenant au total de 3 à 5 atomes de carbone, ou un diéthylaminocarbonyl-méthyle; ou un radical de formule VII:

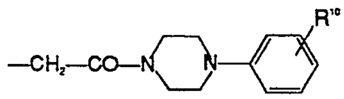

(VII);

$R^4$ représente l'hydrogène; un alkyle en $C_1$-$C_4$; un phénylalkyle dont l'alkyle contient 1 ou 2 atomes de carbone et qui peut éventuellement contenir du chlore;
$R^5$ représente l'hydrogène ou un alcoxy en $C_1$-$C_4$;
$R^6$ représente l'hydrogène ou un phényle;
$R^7$ représente un amino; un pipéridyle-1; un pipérazinyle-1; un morpholinyle-1 ou un alkyl-4 pipérazinyle-1 dont l'alkyle contient de 1 à 4 atomes de carbone;
$R^8$ représente l'hydrogène ou un phényle éventuellement porteur d'un chlore;
$R^9$ représente l'hydrogène; un phénoxy-acétyle ou un radical de formule VI:

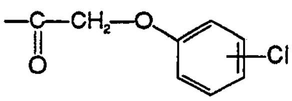

(VI) ; et

$R^{10}$ représente l'hydrogène ou un alcoxy en $C_1$-$C_4$,

ainsi que les sels d'addition acceptables du point de vue physiologique que ces composés forment avec des acides.

2. Pipérazinone-2 substituée selon la revendication 1, caractérisée en ce que:

$R^1$ représente un radical thiényle-2,
$R^2$ représente l'hydrogène et
$R^3$ représente l'hydrogène.

3. [(Méthoxy-2 éthoxy)-4 phényl]-3 pipérazinone-2.

4. Pipérazinones substituées qui répondent à la formule Ia:

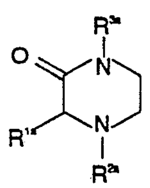

(Ia)

dans laquelle:

$R^{1a}$ représente un radical furyle-2,
$R^{2a}$ représente l'hydrogène ou un radical hydroxy-2 éthyle ou chloro-2 benzyle et
$R^{3a}$ représente l'hydrogène.

5. Pipérazinones substituées répondant à la formule Ib:

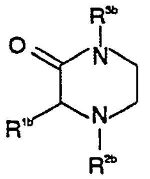

(Ib)

dans laquelle:

$R^{1b}$ représente un radical thiényle-2,
$R^{2b}$ représente un radical allyle ou propargyle et
$R^{3b}$ représente l'hydrogène.

6. Procédé pour préparer les pipérazinones-2 substituées de formule I qui ont été définies dans les revendications 1 à 5, procédé caractérisé en ce qu'on réduit une dihydro-5,6 (1H)-pyrazinone-2 répondant à la formule VIII:

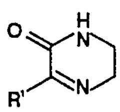

(VIII)

dans laquelle $R^1$ a la signification qui lui a été donnée dans les revendications 1 à 5, de manière à la convertir en la pipérazinone-2 substituée de façon correspondante, qui répond à la formule générale I dans laquelle $R^2$ et $R^3$ représentent chacun l'hydrogène, le cas échéant on fait ensuite réagir celle-ci, de manière connue, avec un agent d'alkylation ou d'acylation répondant à la formule IX:

$$X - R^2 \qquad \text{(IX)}$$

dans laquelle X représente un radical éliminable à l'état d'anion et $R^2$ a la signification qui lui a été donnée dans les revendications 1 à 5, et/ou on fait réagir éventuellement, de manière connue, la pipérazinone de formule I obtenue, dans laquelle $R^3$ représente l'hydrogène, avec un agent d'alkylation répondant à la formule X:

$$X - R^3 \qquad \text{(X)}$$

dans laquelle X représente un radical éliminable à l'état d'anion et $R^3$ a la signification qui lui a été donnée dans les revendications 1 à 5, et, si on le désire, on transforme, de manière connue, la pipérazinone-2 substituée obtenue en un sel d'addition d'acide acceptable du point de vue physiologique.

7. Application d'une pipérazinone-2 substituée ou d'un de ses sels d'addition d'acides acceptables du point de vue physiologique, selon l'une ou plusieurs des revendications 1 à 5, à la fabrication d'un médicament destiné à améliorer la fonction cérébrale.

8. Application d'une pipérazinone-2 substituée, ou d'un de ses sels d'addition d'acides acceptables du point de vue physiologique, selon l'une ou plusieurs des revendications 1 à 5, à la fabrication de médicaments à action nootropique.

**Revendications** pour l'Etat Contractant: AT

1. Procédé pour préparer des pipérazinones substituées répondant à la formule générale I:

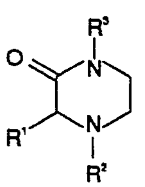

(I)

dans laquelle:

$R^1$ représente un phényle porteur d'un alcoxy en $C_1$-$C_4$, le fluor, un alcoxy-alkyle contenant au total de 2 à 6 atomes de carbone ou un dialkylamino-alcoxy contenant au total de 4 à 8 atomes de carbone; un thiényle-2; un pyridyle-3 ou un radical de formule II:

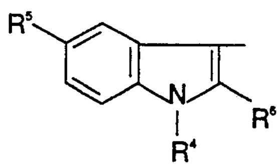

(II);

$R^2$ représente l'hydrogène, un alkyle en $C_1$-$C_4$, éventuellement porteur d'un alcoxycarbonyle contenant au total de 2 à 5 atomes de carbone, ou d'un aminocarbonyle dont l'amino peut lui-même porter un ou deux substituants pris dans l'ensemble constitué par le méthyle et l'éthyle ou fait partie d'un noyau de pipéridine, de pipérazine ou de morpholine; un phénylalkyle dont l'alkyle contient de 1 à 3 atomes de carbone et dont le noyau phényle porte éventuellement du chlore, un alcoxy à un ou deux atomes de carbone ou un hydroxy; un alcanoyle en $C_1$-$C_4$, éventuellement porteur de chlore; un nicotinoyle; un benzoyle éventuellement porteur d'un alcoxy à un ou deux atomes de carbone, d'un nitro ou d'un amino; un aminosulfonyle; un aminosulfonyle dont l'azote porte un ou deux alkyles contenant chacun de 1 à 4 atomes de carbone, ou un aminosulfonyle dont le radical amino fait partie d'un cycle de pipéridine, de pipérazine, de morpholine ou d'alkyl-4 pipérazine, l'alkyle en 4 de ce dernier contenant de 1 à 4 atomes de carbone; un radical de formule III:

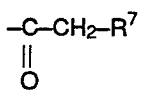

(III);

un radical de formule IV:

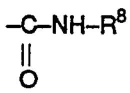

(IV);

ou un radical de formule V:

$$-CH_2-CH_2-OR^9 \qquad (V);$$

$R^3$ représente l'hydrogène; un alkyle en $C_1$-$C_4$; un alcoxycarbonylméthyle contenant au total de 3 à 6 atomes de carbone; un aminocarbonylméthyle; un aminocarbonylméthyle mono ou di-substitué à l'azote et contenant au total de 3 à 5 atomes de carbone, ou un diéthylaminocarbonyl-méthyle; ou un radical de formule VII:

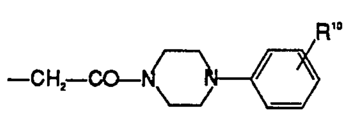

(VII);

$R^4$ représente l'hydrogène; un alkyle en $C_1$-$C_4$; un phénylalkyle dont l'alkyle contient 1 ou 2 atomes de carbone et qui peut éventuellement contenir du chlore;

$R^5$ représente l'hydrogène ou un alcoxy en $C_1$-$C_4$;

$R^6$ représente l'hydrogène ou un phényle;

$R^7$ représente un amino un pipéridyle-1 ; un pipérazinyle-1; un morpholinyle-1 ou un alkyl-4 pipérazinyle-1 dont l'alkyle contient de 1 à 4 atomes de carbone;

$R^8$ représente l'hydrogène ou un phényle éventuellement porteur d'un chlore;
$R^9$ représente l'hydrogène; un phénoxy-acétyle ou un radical de formule VI:

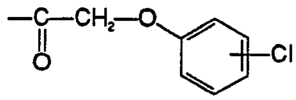

(VI) ; et

$R^{10}$ représente l'hydrogène ou un alcoxy en $C_1$-$C_4$, ainsi que des sels d'addition acceptables du point de vue physiologique que ces composés forment avec des acides, procédé caractérisé en ce qu'on réduit de manière connue une dihydro-5,6 (1H)-pyrazinone-2 répondant à la formule VIII:

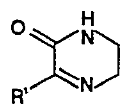

de manière à la convertir en la pipérazinone-2 substituée de façon correspondante, qui répond à la formule générale I dans laquelle $R^2$ et $R^3$ représentent chacun l'hydrogène, après quoi on fait éventuellement réagir celle-ci, de manière connue, avec un agent d'alkylation ou d'acylation répondant à la formule IX:

$$X{-}R^2 \qquad (IX)$$

dans laquelle X représente un radical éliminable à l'état d'anion, et/ou on fait éventuellement réagir, de manière connue, la pipérazinone-2 obtenue, qui répond à la formule I dans laquelle $R^3$ représente l'hydrogène, avec un agent d'alkylation répondant à la formule X:

$$X{-}R^3 \qquad (X)$$

dans laquelle X représente un radical éliminable à l'état d'anion, et, si on le désire, on transforme de manière connue la pipérazinone-2 substituée obtenue en un sel d'addition d'acide acceptable du point de vue physiologique.

2. Procédé selon la revendication 1 caractérisé en ce qu'on choisit les corps de départ de telle façon qu'il se forme un composé de formule I dans lequel:

$R^1$ représente un radical thiényle-2,
$R^2$ représente l'hydrogène et
$R^3$ représente l'hydrogène.

3. Procédé de préparation de la [(méthoxy-2 éthoxy)-4 phényl]-3 pipérazinone-2, procédé caractérisé en ce qu'on hydrogène la [(méthoxy-2 éthoxy)-4 phényl]-3 dihydro-5,6 (1H)-pyrazinone-2.

4. Procédé pour préparer des pipérazinones substituées répondant à la formule Ia:

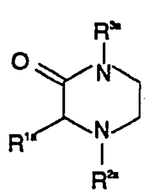

(Ia)

dans laquelle:

$R^{1a}$ représente un radical furyle-2,
$R^{2a}$ représente l'hydrogène ou un radical hydroxy-2 éthyle ou chloro-2 benzyle et

$R^{3a}$ représente l'hydrogène,

procédé caractérisé en ce qu'on hydrogène la (furyl-2)-3 dihydro-5,6 (1H)-pyrazinone-2, puis on fait éventuellement réagir, de manière connue, avec un agent d'alkylation répondant à la formule IXa:

$X{-}R^{2a}$ (IXa)

dans laquelle X représente un radical éliminable à l'état d'anion.

5. Procédé pour préparer des pipérazinones substituées répondant à la formule Ib:

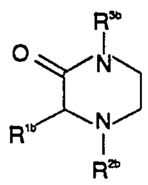

(Ib)

dans laquelle:

$R^{1b}$ représente un radical thiényle-2,
$R^{2b}$ représente un radical allyle ou propargyle et
$R^{3b}$ représente l'hydrogène,

procédé caractérisé en ce qu'on hydrogène la (thiényl-2)-3 dihydro-5,6 (1H)-pyrazinone-2, et on fait ensuite réagir, de manière connue, avec un agent d'alkylation répondant à la formule IXb:

$X{-}R^{2b}$ (IXb)

dans laquelle X représente un radical éliminable à l'état d'anion.

6. Application d'une pipérazinone-2 substituée qui a été préparée par le procédé des revendications 1 à 5, ou d'un de ses sels d'addition d'acides acceptables du point de vue physiologique, à la fabrication d'un médicament destiné à améliorer la fonction cérébrale.

7. Application d'une pipérazinone-2 substituée qui a été préparée par le procédé des revendications 1 à 5, ou d'un de ses sels d'addition d'acides acceptables du point de vue physiologique, à la fabrication de médicaments à action nootropique.